# EUROPEAN PATENT APPLICATION

(11) **EP 2 450 451 A1**
(43) Date of publication of application: **09.05.2012**
(21) Application number: 10794058.7
(22) Date of filing: 24.06.2010
(51) Int. Cl.: C12P 13/04, C12N 15/09

(54) **METHOD FOR RACEMIZING OPTICALLY ACTIVE -AMINO ACIDS**

(30) Priority: 29.06.2009 JP 2009154287
(71) Applicant: Mitsubishi Gas Chemical Company, Inc., Tokyo 100-8324 (JP)
(72) Inventor: INOUE Atsushi, Niigata-shi Niigata 950-3112 (JP); TANAKA Yusuke, Niigata-shi Niigata 950-3112 (JP)
(74) Representative: Müller-Boré & Partner Patentanwälte
(86) International application number: PCT/JP2010/060744
(87) International publication number: WO 2011/001889

(57) **Abstract**

The present invention relates to a method for racemizing an optically active α-amino acid with a viable bacterial cell producing γ-aminobutyric acid aminotransferase or a processed product thereof. According to the present invention, racemic α-amino acids of high quality can be manufactured inexpensively.

SEQUENCE LISTING

<110> Mitsubishi Gas Chemical Company, Inc.

<120> Method for racemization of optical active alpha-amino acids

<130> M 5567EU

<140> 10794058.7
<141> 24.06.2010

<150> 2009-154287
<151> 29.06.2009

<160> 17

<170> PatentIn version 3.5

<210> 1
<211> 444
<212> PRT
<213> Leuconostoc mesenteroides subsp. Mesenteroides ATCC8293

<400> 1

Met Ser Lys Asn Lys Glu Ile Leu Glu Glu Glu Lys Ile His Leu Ala
1               5                   10                  15

Asn Ala Thr Arg Val Lys Tyr Phe Asp Ile Val Ile Asp His Gly Lys
            20                  25                  30

Gly Ala Ile Ile Thr Asp Ala Asp Gly His Glu Tyr Ile Asp Leu Leu
        35                  40                  45

Ala Ser Ala Ser Ala Thr Asn Thr Gly His Ser His Pro Lys Val Val
    50                  55                  60

Glu Ala Ile Thr Ser Gln Ala Gln Lys Leu Ile Gln Tyr Thr Pro Ala
65                  70                  75                  80

Tyr Phe Ala Asn Thr Val Thr Ala Lys Leu Ala Asp Arg Leu Val Asn
                85                  90                  95

Val Ala Pro Gly Asn Phe Gln Lys Gln Val Ala Phe Gly Asn Ser Gly
            100                 105                 110

Ser Asp Ala Asn Asp Ala Ile Ile Lys Phe Ala Arg Gly Tyr Thr Gly
        115                 120                 125

Arg Gln Tyr Val Val Ser Phe Thr Gly Ala Tyr His Gly Ser Thr Tyr
    130                 135                 140

Gly Ser Leu Ser Ile Ser Gly Val Ser Leu Asn Met Thr Arg Lys Ile
145                 150                 155                 160

Gly Pro Leu Leu Pro Asn Thr Val Lys Val Pro Phe Pro Asp Gln Thr
                165                 170                 175

Gln Arg Ile Ala Gly Glu Ser Asp Asn Asp Phe Ser Glu Arg Leu Phe

180                 185                 190

Lys Gln Phe Lys Leu Pro Phe Glu Thr Tyr Leu Pro Ala Asp Glu Val
        195                 200                 205

Ala Leu Val Ile Ile Glu Pro Ile Gln Gly Asp Gly Gly Ile Ile Lys
    210                 215                 220

Ala Pro Gln His Tyr Val Glu Leu Val Tyr Glu Phe Thr Arg Glu His
225                 230                 235                 240

Gly Ile Val Phe Ala Val Asp Glu Val Asn Gln Gly Leu Gly Arg Thr
                245                 250                 255

Gly Lys Met Trp Ser Ile Asp His Phe Gly Ile Ala Pro Asp Leu Met
            260                 265                 270

Ser Val Gly Lys Ser Ile Ala Ser Gly Leu Pro Leu Ser Ala Val Ile
        275                 280                 285

Gly Arg Lys Glu Ile Met Asp Ser Leu Gln Ala Pro Ala Asn Val Tyr
    290                 295                 300

Thr Thr Ala Gly Asn Pro Val Thr Ala Ala Ala Ala Leu Ala Thr Leu
305                 310                 315                 320

Asp Val Ile Glu Glu Glu Asn Leu Val Ala Arg Ser Arg Gln Leu Gly
                325                 330                 335

Glu Leu Ala Lys Ala Phe Phe Glu Ser Ala Ala Lys Arg Tyr Ala Phe
            340                 345                 350

Ile Gly Asp Val Arg Leu Tyr Gly Leu Asn Gly Gly Ile Asp Ile Ile
        355                 360                 365

Asn Asp His Gly Glu Pro Asp Asn Glu Ala Thr Asn Lys Leu Ile Tyr
    370                 375                 380

Arg Ile Phe Glu Leu Gly Ala Ile Met Ile Ser Leu Arg Gly Asn Thr
385                 390                 395                 400

Leu Arg Phe Gln Pro Pro Leu Val Ile Gln Glu Glu Glu Leu Gln Arg
                405                 410                 415

Ala Phe Glu Ile Leu Glu Lys Ala Phe Ser Asp Leu Ala Ser Gly Lys
            420                 425                 430

Ile Ala Leu Pro Lys Thr Asp Asn His Ile Gly Trp
        435                 440

<210>  2
<211>  445
<212>  PRT

<213>  Staphylococcus saprophyticus subsp[?]D Saprophyticus ATCC 15305

<400>  2

Met Asn Lys Ser Glu Gln Leu Ile Asn Glu Asp Ser Gln Tyr Phe Ala
1               5                   10                  15

Lys Pro Gly Arg Ile Lys Tyr Tyr Pro Leu Ala Ile Ser His Gly Tyr
            20                  25                  30

Gly Ala Thr Leu Val Asp Val Glu Gly Asn Thr Tyr Ile Asp Leu Leu
        35                  40                  45

Ser Ser Ala Ser Ser Gln Asn Val Gly His Ala Pro Glu Gln Val Thr
    50                  55                  60

Glu Ala Ile Lys Ala Gln Val Asp Gln Phe Ile His Tyr Thr Pro Ala
65                  70                  75                  80

Tyr Met Tyr His Glu Pro Leu Val Lys Leu Ala Lys Lys Leu Cys Glu
                85                  90                  95

Ile Ser Pro Gly Gln Tyr Glu Lys Arg Val Leu Phe Gly Leu Ser Gly
            100                 105                 110

Ser Asp Ala Asn Asp Gly Ile Ile Lys Leu Ala Arg Ala Tyr Thr Gly
        115                 120                 125

Arg Pro Tyr Ile Ile Ser Phe Val Asn Ala Tyr His Gly Ser Thr Tyr
    130                 135                 140

Gly Ser Leu Ser Met Ser Ala Ile Ser Leu Asn Met Arg Lys Tyr Tyr
145                 150                 155                 160

Gly Pro Met Leu Pro Gly Phe Tyr His Ile Pro Phe Pro Asp Asn Tyr
                165                 170                 175

Arg Gly Met Phe Gly Ser Asn Gln Pro Asn Thr Val Ser Glu Tyr Leu
            180                 185                 190

Ala Pro Leu Lys Glu Met Phe Glu Lys Tyr Val Pro Ala Glu Glu Val
        195                 200                 205

Ala Cys Ile Met Ile Glu Thr Ile Gln Gly Asp Gly Gly Leu Leu Glu
    210                 215                 220

Pro Val Asp Gly Tyr Phe Glu Ala Leu Gln Asp Leu Cys Lys Glu His
225                 230                 235                 240

Gly Ile Leu Leu Ala Val Asp Asp Ile Gln Gln Gly Leu Gly Arg Thr
                245                 250                 255

Gly Ser Trp Ser Ser Val Ser His Tyr Asn Ile Glu Pro Asp Leu Ile
            260                 265                 270

Thr Phe Gly Lys Ser Leu Ala Gly Gly Leu Pro Met Ser Ala Ile Val
        275                 280                 285

Gly Arg Ser Glu Ile Met Asp Tyr Leu Glu Ala Pro Ala His Leu Phe
    290                 295                 300

Thr Thr Gly Ala Asn Pro Val Ser Cys Glu Ala Ala Leu Ala Thr Ile
305                 310                 315                 320

Ala Met Ile Glu Asp Gln Asn Leu Leu Gln Ala Ser Thr Asp Lys Gly
                325                 330                 335

Asn Tyr Val Arg Lys Arg Ile Asp Ala Trp Thr Glu Gln Phe Asn Thr
            340                 345                 350

Val Gly Asn Val Arg Gly Lys Gly Leu Ser Ile Gly Ile Asp Ile Val
        355                 360                 365

Ser Asp Lys Ile Asn Lys Thr Arg Ala Ser Glu Ala Ala Leu Lys Ile
    370                 375                 380

Cys Asn Arg Cys Phe Asp Asn Gly Val Val Ile Ile Ala Val Ala Gly
385                 390                 395                 400

Asn Val Leu Arg Phe Gln Pro Pro Leu Val Ile Thr Tyr Glu Gln Leu
                405                 410                 415

Asp Thr Ala Leu Asn Val Leu Glu Ala Ala Ile Ser Asp Phe Glu Ser
            420                 425                 430

Gly Lys Leu Asp Asn Tyr Asp Thr Ala Gly Gln Gly Trp
        435                 440                 445

<210>  3
<211>  450
<212>  PRT
<213>  Lactobacillus fermentum IFO 3956

<400>  3

Met Glu Glu Lys Lys Ser Asn Gln Gln Met Leu Ala Ala Glu Glu Glu
1               5                   10                  15

Ala Phe Ser Ser Ala Ala Arg Ile Lys Tyr Tyr Asp Ile Val Ile Asp
            20                  25                  30

Lys Gly Arg Gly Ala Ile Ile Thr Asp Val Glu Gly His Asp Tyr Ile
        35                  40                  45

Asp Leu Leu Ala Ser Ala Ser Ser Thr Asn Thr Gly His Ala His Pro
    50                  55                  60

Lys Val Val Lys Ala Ile Ala Asp Gln Ala Ala Arg Leu Ile Gln Tyr

65                  70                  75                  80

Thr Pro Ala Tyr Phe Ala Asn Ser Gln Ala Ala Arg Leu Ala Pro Arg
                85                  90                  95

Leu Ala Ala Leu Ala Pro Ile Ser Gly Pro Val Glu Val Ala Trp Gly
            100                 105                 110

Asn Ser Gly Ser Asp Ala Asn Asp Ala Ile Ile Lys Phe Ala Arg Ala
        115                 120                 125

Ala Thr Gly Arg Gln Tyr Ile Val Ser Phe Thr Gly Ala Tyr His Gly
    130                 135                 140

Ser Thr Tyr Gly Ser Met Thr Ile Ser Ser Val Thr Leu Asn Met Ser
145                 150                 155                 160

Arg Lys Met Gly Pro Leu Leu Pro Gly Val Val Lys Val Pro Phe Pro
                165                 170                 175

Ser Pro Trp Asp Lys Leu Ala Gly Glu Ser Asp Asp Gln Phe Val Asp
            180                 185                 190

Arg Met Tyr Lys Ala Phe Leu Gln Pro Phe Glu Thr Tyr Leu Pro Ala
        195                 200                 205

Glu Glu Val Ala Ala Ile Val Ile Glu Pro Ile Gln Gly Asp Gly Gly
    210                 215                 220

Ile Ile Lys Val Pro Gln Ala Tyr Met Glu Arg Ile Tyr Ala Phe Ala
225                 230                 235                 240

Lys Gln Asn Gly Ile Leu Phe Ala Ile Asp Glu Val Asn Gln Gly Met
                245                 250                 255

Gly Arg Thr Gly Lys Trp Trp Ser Ile Gln His Phe Pro Gly Ile Glu
            260                 265                 270

Pro Asp Leu Met Ser Val Gly Lys Ser Leu Ala Ser Gly Met Pro Leu
        275                 280                 285

Ser Ala Val Val Gly Arg Lys Gly Ile Met Glu Ala Leu Gly Pro Ala
    290                 295                 300

Ala Asn Val Tyr Thr Thr Ala Gly Asn Pro Val Thr Ala Ala Ala Ala
305                 310                 315                 320

Asn Ala Thr Leu Asp Val Ile Glu Glu Glu His Leu Val Glu Arg Ser
                325                 330                 335

Ala Lys Leu Gly Gln Lys Ala Lys Asp Phe Phe Asp Gln Glu Gln Ala
            340                 345                 350

Lys Tyr Asp Phe Val Gly Asp Ala Arg Leu Tyr Gly Leu Asn Gly Gly
        355                 360                 365

Ile Ser Ile Val Asp Pro Ala Thr Gly Lys Gly Asp Val Glu Ala Thr
    370                 375                 380

Thr Lys Leu Met Tyr Arg Ile Phe Glu Leu Gly Ala Ile Ile Ile Ser
385                 390                 395                 400

Leu Arg Gly Asn Ile Leu Arg Phe Gln Pro Pro Leu Val Ile Thr Glu
                405                 410                 415

Glu Gln Leu Asp Lys Ala Phe Ala Ile Ile Asp Gln Ala Phe Ala Glu
            420                 425                 430

Leu Ala Ala Gly Lys Leu Ser Leu Pro Ser Asn Ala Glu Glu Leu Gly
        435                 440                 445

Trp Glu
    450

<210>  4
<211>  456
<212>  PRT
<213>  Lactobacillus reuteri DSM 20016

<400>  4

Met Asp Lys Met Leu Lys Thr Lys Leu Lys Gln Lys Lys Thr Asn Arg
1               5                   10                  15

Asp Val Leu Leu Glu Glu Lys Asn Ala Phe Ala Ser Ala Ala Arg Ile
            20                  25                  30

Lys Tyr Tyr Asp Ile Val Leu Asp His Gly Arg Gly Ala Met Val Thr
        35                  40                  45

Asp Ile Glu Gly Asn Asp Tyr Ile Asp Leu Leu Ala Ser Ala Ser Ser
    50                  55                  60

Thr Asn Thr Gly His Ala His Pro Arg Val Val Gln Ala Ile Gln Glu
65                  70                  75                  80

Gln Ala Ala Lys Met Ile Gln Tyr Thr Pro Ala Tyr Phe Ala Asn Ser
                85                  90                  95

Gln Ala Ala Arg Leu Ala Pro Arg Leu Ala Glu Leu Ala Pro Ile Ser
            100                 105                 110

Gly Pro Val Glu Met Val Trp Gly Asn Ser Gly Ser Asp Ala Asn Asp
        115                 120                 125

Ala Ile Ile Lys Phe Ala Arg Ala Tyr Thr Gly Arg Gln Tyr Ile Ile
    130                 135                 140

Ser Phe Thr Gly Ala Tyr His Gly Ser Thr Tyr Gly Ser Met Ser Leu
145                 150                 155                 160

Ser Gly Val Ser Leu Asn Met Thr Arg Lys Met Gly Pro Met Leu Pro
                165                 170                 175

Gly Val Val Lys Val Pro Phe Pro Ser Pro Trp Glu Arg Leu Thr Asn
            180                 185                 190

Glu Ser Asp Glu Ala Phe Val Glu Arg Met Phe His Gln Phe Met Leu
        195                 200                 205

Pro Phe Glu Thr Tyr Leu Pro Val Asp Glu Val Ala Ala Ile Leu Ile
    210                 215                 220

Glu Pro Ile Gln Gly Asp Gly Gly Ile Val Lys Thr Pro Pro Ala Tyr
225                 230                 235                 240

Met Gln Lys Val Tyr Glu Phe Ala Lys Ala Asn Gly Ile Leu Phe Ala
                245                 250                 255

Val Asp Glu Val Asn Gln Gly Met Gly Arg Thr Gly Lys Trp Trp Ser
            260                 265                 270

Ile Gln His Phe Gly Ile Glu Pro Asp Leu Met Ser Val Gly Lys Ser
        275                 280                 285

Leu Ala Ser Gly Met Pro Leu Ser Ala Met Ile Gly Arg Lys Glu Ile
    290                 295                 300

Leu Glu Ser Leu Gly Ala Pro Ala Asn Val Tyr Thr Thr Ala Gly Asn
305                 310                 315                 320

Pro Val Thr Thr Ala Ala Ala Asn Ala Thr Leu Asp Val Ile Gln Asn
                325                 330                 335

Glu His Leu Leu Glu Arg Ser Gln Arg Leu Gly Lys Lys Ala Ala Glu
            340                 345                 350

Phe Phe Lys Leu Glu Gln Gln Lys Tyr Pro Phe Val Gly Asp Val Arg
        355                 360                 365

Met Tyr Gly Leu Asp Gly Gly Ile Asp Ile Ile Asp Pro Val Thr Gly
    370                 375                 380

Lys Gly Asp Thr Glu Ala Thr Thr Lys Leu Met Tyr Arg Ile Phe Glu
385                 390                 395                 400

Leu Gly Ala Ile Ile Ile Ser Leu Arg Gly Ser Ile Leu Arg Phe Gln
                405                 410                 415

Pro Pro Leu Val Ile Thr Glu Lys Glu Leu Asp Gln Ala Phe Ala Met
        420                 425                 430

Ile Asp Gln Ala Phe Ala Glu Leu Ala Ala Gly Lys Leu Ala Ala Pro
    435                 440                 445

Ala Asn Ala Glu Glu Leu Gly Trp
    450                 455

<210>  5
<211>  445
<212>  PRT
<213>  Staphyrococcus epidermidis ATCC 12228

<400>  5

Met Ser Lys Val Asn His Leu Ile Asp Glu Asp Glu Arg Tyr Phe Ala
1               5                   10                  15

His Ser Gly Arg Ile Lys Tyr Tyr Pro Leu Val Ile Asp His Gly Tyr
            20                  25                  30

Gly Ala Thr Leu Ile Asp Ile Asp Gly Lys Ser Tyr Ile Asp Leu Leu
        35                  40                  45

Ala Ser Ala Ser Ser Gln Asn Val Gly His Ala Pro Lys Pro Val Val
    50                  55                  60

Glu Ala Ile Lys Asn Gln Thr Glu Lys Phe Ile His Tyr Thr Pro Ala
65                  70                  75                  80

Tyr Met Tyr His Glu Pro Leu Val Arg Leu Ser Lys Lys Leu Cys Asp
                85                  90                  95

Ile Ala Pro Gly Asn Tyr Glu Lys Arg Val Thr Phe Gly Leu Ser Gly
            100                 105                 110

Ser Asp Ala Asn Asp Gly Ile Ile Lys Phe Ala Arg Ala Tyr Thr Gly
        115                 120                 125

Arg Pro Tyr Ile Ile Ser Phe Thr Asn Ala Tyr His Gly Ser Thr Phe
    130                 135                 140

Gly Ser Leu Ser Met Ser Ser Ile Ser Leu Asn Met Arg Lys Arg Tyr
145                 150                 155                 160

Gly Pro Leu Leu Asn Gly Phe Tyr His Ile Pro Phe Pro Asp Lys Tyr
                165                 170                 175

Arg Gly Met Phe Glu Gln Ala Lys Pro Asn Thr Val Glu Glu Tyr Leu
            180                 185                 190

Ala Pro Leu Lys Glu Met Phe Thr Lys Tyr Val Pro Ala Glu Glu Val
        195                 200                 205

Ala Cys Ile Val Val Glu Thr Ile Gln Gly Asp Gly Gly Leu Leu Glu
    210                 215                 220

Pro Val Pro Gly Tyr Phe Glu Ala Leu Gln Glu Leu Cys His Ala His
225                 230                 235                 240

Asn Ile Leu Ile Ala Val Asp Asp Ile Gln Gln Gly Leu Gly Arg Thr
                245                 250                 255

Gly Lys Trp Ser Ser Val Asp His Tyr His Phe Thr Pro Asp Leu Met
            260                 265                 270

Thr Phe Gly Lys Ser Leu Ala Gly Gly Leu Pro Met Ser Ala Ile Val
        275                 280                 285

Gly Arg Lys Glu Ile Met Glu Ser Leu Glu Ala Pro Ala His Leu Phe
    290                 295                 300

Thr Thr Gly Ala Asn Pro Val Ser Cys Glu Ala Ala Leu Ala Thr Ile
305                 310                 315                 320

Lys Met Ile Glu Asp Glu Asp Leu Leu Asn Ala Ser Trp Lys Lys Gly
                325                 330                 335

Ser Tyr Val Arg Lys Arg Ile Asp Pro Trp Ile Glu Arg Tyr Gln Tyr
            340                 345                 350

Val Gly Asp Val Arg Gly Ile Gly Leu Ser Ile Gly Ile Asp Ile Val
        355                 360                 365

Ser Asn Lys Ile Glu Lys Thr Arg Asp Ser Glu Ala Ala Leu Lys Ile
    370                 375                 380

Cys Asn Tyr Cys Phe Glu Asn Gly Val Ile Ile Ile Ala Val Ala Gly
385                 390                 395                 400

Asn Val Leu Arg Phe Gln Pro Pro Leu Val Ile Thr Tyr Lys Gln Leu
                405                 410                 415

Asp Lys Ala Leu Asp Thr Ile Glu Glu Ala Leu Glu Lys Leu Glu Arg
            420                 425                 430

Gly Glu Leu Asn Gln Tyr Asp Ile Ser Gly Gln Gly Trp
        435                 440                 445

<210>  6
<211>  58
<212>  DNA
<213>  Artificial Sequence

<220>
<223>  primer

<400>  6

ctaaaaaagc ttgtaggagg tgtagaaaat gtcaaaaaat aaagagattt tagaagaa 58

<210> 7
<211> 22
<212> DNA
<213> Artificial Sequence

<220>
<223> primer

<400> 7
cgtatgtcta gaattcctaa tc 22

<210> 8
<211> 57
<212> DNA
<213> Artificial Sequence

<220>
<223> primer

<400> 8
attttaagct tttaggaggc atagattatg aacaaatcag agcaacttat aaatgag 57

<210> 9
<211> 24
<212> DNA
<213> Artificial Sequence

<220>
<223> primer

<400> 9
gtttatttaa attctagaag gttg 24

<210> 10
<211> 35
<212> DNA
<213> Artificial Sequence

<220>
<223> primer

<400> 10
gagacactgc catatggagg agaaaaaaag taacc 35

<210> 11
<211> 33
<212> DNA
<213> Artificial Sequence

<220>
<223> primer

<400> 11
ctgaattctt attcccaacc cagctcttct gcg 33

<210> 12
<211> 35
<212> DNA
<213> Artificial Sequence

<220>
<223> primer

<400>  12
gaagatggac catatgttaa aaacgaaact gaagc                                35

<210>  13
<211>  37
<212>  DNA
<213>  Artificial Sequence

<220>
<223>  primer

<400>  13
gattggtcga cttaccaacc taactcttct gcatttg                              37

<210>  14
<211>  32
<212>  DNA
<213>  Artificial Sequence

<220>
<223>  primer

<400>  14
gagggaacca tatgagtaag gttaatcatt tg                                   32

<210>  15
<211>  32
<212>  DNA
<213>  Artificial Sequence

<220>
<223>  primer

<400>  15
cagaattctt accaaccttg accactaatg tc                                   32

<210>  16
<211>  30
<212>  DNA
<213>  Artificial Sequence

<220>
<223>  primer

<400>  16
cacaggaaac agcatatggc catgattacg                                      30

<210>  17
<211>  30
<212>  DNA
<213>  Artificial Sequence

<220>
<223>  primer

<400>  17
cgtaatcatg gccatatgct gtttcctgtg                                      30

SEQUENCE LISTING

<110> Mitsubishi Gas Chemical Company, Inc.

<120> Method for racemization of optical active alpha-amino acids

<130> M 5567EU

<140> 10794058.7
<141> 24.06.2010

<150> 2009-154287
<151> 29.06.2009

<160> 26

<170> PatentIn version 3.5

<210> 1
<211> 444
<212> PRT
<213> Leuconostoc mesenteroides subsp. Mesenteroides ATCC8293

<400> 1

Met Ser Lys Asn Lys Glu Ile Leu Glu Glu Glu Lys Ile His Leu Ala
1               5                   10                  15

Asn Ala Thr Arg Val Lys Tyr Phe Asp Ile Val Ile Asp His Gly Lys
            20                  25                  30

Gly Ala Ile Ile Thr Asp Ala Asp Gly His Glu Tyr Ile Asp Leu Leu
        35                  40                  45

Ala Ser Ala Ser Ala Thr Asn Thr Gly His Ser His Pro Lys Val Val
    50                  55                  60

Glu Ala Ile Thr Ser Gln Ala Gln Lys Leu Ile Gln Tyr Thr Pro Ala
65                  70                  75                  80

Tyr Phe Ala Asn Thr Val Thr Ala Lys Leu Ala Asp Arg Leu Val Asn
                85                  90                  95

Val Ala Pro Gly Asn Phe Gln Lys Gln Val Ala Phe Gly Asn Ser Gly
            100                 105                 110

Ser Asp Ala Asn Asp Ala Ile Ile Lys Phe Ala Arg Gly Tyr Thr Gly
        115                 120                 125

Arg Gln Tyr Val Val Ser Phe Thr Gly Ala Tyr His Gly Ser Thr Tyr
    130                 135                 140

Gly Ser Leu Ser Ile Ser Gly Val Ser Leu Asn Met Thr Arg Lys Ile
145                 150                 155                 160

Gly Pro Leu Leu Pro Asn Thr Val Lys Val Pro Phe Pro Asp Gln Thr
                165                 170                 175

Gln Arg Ile Ala Gly Glu Ser Asp Asn Asp Phe Ser Glu Arg Leu Phe

            180                 185                 190

Lys Gln Phe Lys Leu Pro Phe Glu Thr Tyr Leu Pro Ala Asp Glu Val
        195                 200                 205

Ala Leu Val Ile Ile Glu Pro Ile Gln Gly Asp Gly Gly Ile Ile Lys
    210                 215                 220

Ala Pro Gln His Tyr Val Glu Leu Val Tyr Glu Phe Thr Arg Glu His
225                 230                 235                 240

Gly Ile Val Phe Ala Val Asp Glu Val Asn Gln Gly Leu Gly Arg Thr
                245                 250                 255

Gly Lys Met Trp Ser Ile Asp His Phe Gly Ile Ala Pro Asp Leu Met
            260                 265                 270

Ser Val Gly Lys Ser Ile Ala Ser Gly Leu Pro Leu Ser Ala Val Ile
        275                 280                 285

Gly Arg Lys Glu Ile Met Asp Ser Leu Gln Ala Pro Ala Asn Val Tyr
    290                 295                 300

Thr Thr Ala Gly Asn Pro Val Thr Ala Ala Ala Ala Leu Ala Thr Leu
305                 310                 315                 320

Asp Val Ile Glu Glu Glu Asn Leu Val Ala Arg Ser Arg Gln Leu Gly
                325                 330                 335

Glu Leu Ala Lys Ala Phe Phe Glu Ser Ala Ala Lys Arg Tyr Ala Phe
            340                 345                 350

Ile Gly Asp Val Arg Leu Tyr Gly Leu Asn Gly Gly Ile Asp Ile Ile
        355                 360                 365

Asn Asp His Gly Glu Pro Asp Asn Glu Ala Thr Asn Lys Leu Ile Tyr
    370                 375                 380

Arg Ile Phe Glu Leu Gly Ala Ile Met Ile Ser Leu Arg Gly Asn Thr
385                 390                 395                 400

Leu Arg Phe Gln Pro Pro Leu Val Ile Gln Glu Glu Glu Leu Gln Arg
                405                 410                 415

Ala Phe Glu Ile Leu Glu Lys Ala Phe Ser Asp Leu Ala Ser Gly Lys
            420                 425                 430

Ile Ala Leu Pro Lys Thr Asp Asn His Ile Gly Trp
        435                 440

<210> 2
<211> 445
<212> PRT

<213>  Staphylococcus saprophyticus subsp[?][?] Saprophyticus ATCC 15305

<400>  2

Met Asn Lys Ser Glu Gln Leu Ile Asn Glu Asp Ser Gln Tyr Phe Ala
1               5                   10                  15

Lys Pro Gly Arg Ile Lys Tyr Tyr Pro Leu Ala Ile Ser His Gly Tyr
            20                  25                  30

Gly Ala Thr Leu Val Asp Val Glu Gly Asn Thr Tyr Ile Asp Leu Leu
        35                  40                  45

Ser Ser Ala Ser Ser Gln Asn Val Gly His Ala Pro Glu Gln Val Thr
    50                  55                  60

Glu Ala Ile Lys Ala Gln Val Asp Gln Phe Ile His Tyr Thr Pro Ala
65                  70                  75                  80

Tyr Met Tyr His Glu Pro Leu Val Lys Leu Ala Lys Lys Leu Cys Glu
                85                  90                  95

Ile Ser Pro Gly Gln Tyr Glu Lys Arg Val Leu Phe Gly Leu Ser Gly
            100                 105                 110

Ser Asp Ala Asn Asp Gly Ile Ile Lys Leu Ala Arg Ala Tyr Thr Gly
        115                 120                 125

Arg Pro Tyr Ile Ile Ser Phe Val Asn Ala Tyr His Gly Ser Thr Tyr
    130                 135                 140

Gly Ser Leu Ser Met Ser Ala Ile Ser Leu Asn Met Arg Lys Tyr Tyr
145                 150                 155                 160

Gly Pro Met Leu Pro Gly Phe Tyr His Ile Pro Phe Pro Asp Asn Tyr
                165                 170                 175

Arg Gly Met Phe Gly Ser Asn Gln Pro Asn Thr Val Ser Glu Tyr Leu
            180                 185                 190

Ala Pro Leu Lys Glu Met Phe Glu Lys Tyr Val Pro Ala Glu Glu Val
        195                 200                 205

Ala Cys Ile Met Ile Glu Thr Ile Gln Gly Asp Gly Gly Leu Leu Glu
    210                 215                 220

Pro Val Asp Gly Tyr Phe Glu Ala Leu Gln Asp Leu Cys Lys Glu His
225                 230                 235                 240

Gly Ile Leu Leu Ala Val Asp Asp Ile Gln Gln Gly Leu Gly Arg Thr
                245                 250                 255

Gly Ser Trp Ser Ser Val Ser His Tyr Asn Ile Glu Pro Asp Leu Ile
            260                 265                 270

Thr Phe Gly Lys Ser Leu Ala Gly Gly Leu Pro Met Ser Ala Ile Val
        275                 280                 285

Gly Arg Ser Glu Ile Met Asp Tyr Leu Glu Ala Pro Ala His Leu Phe
    290                 295                 300

Thr Thr Gly Ala Asn Pro Val Ser Cys Glu Ala Ala Leu Ala Thr Ile
305                 310                 315                 320

Ala Met Ile Glu Asp Gln Asn Leu Leu Gln Ala Ser Thr Asp Lys Gly
                325                 330                 335

Asn Tyr Val Arg Lys Arg Ile Asp Ala Trp Thr Glu Gln Phe Asn Thr
            340                 345                 350

Val Gly Asn Val Arg Gly Lys Gly Leu Ser Ile Gly Ile Asp Ile Val
        355                 360                 365

Ser Asp Lys Ile Asn Lys Thr Arg Ala Ser Glu Ala Ala Leu Lys Ile
    370                 375                 380

Cys Asn Arg Cys Phe Asp Asn Gly Val Val Ile Ile Ala Val Ala Gly
385                 390                 395                 400

Asn Val Leu Arg Phe Gln Pro Pro Leu Val Ile Thr Tyr Glu Gln Leu
                405                 410                 415

Asp Thr Ala Leu Asn Val Leu Glu Ala Ala Ile Ser Asp Phe Glu Ser
            420                 425                 430

Gly Lys Leu Asp Asn Tyr Asp Thr Ala Gly Gln Gly Trp
        435                 440                 445

<210>  3
<211>  450
<212>  PRT
<213>  Lactobacillus fermentum IFO 3956

<400>  3

Met Glu Glu Lys Lys Ser Asn Gln Gln Met Leu Ala Ala Glu Glu Glu
1               5                   10                  15

Ala Phe Ser Ser Ala Ala Arg Ile Lys Tyr Tyr Asp Ile Val Ile Asp
            20                  25                  30

Lys Gly Arg Gly Ala Ile Ile Thr Asp Val Glu Gly His Asp Tyr Ile
        35                  40                  45

Asp Leu Leu Ala Ser Ala Ser Ser Thr Asn Thr Gly His Ala His Pro
    50                  55                  60

Lys Val Val Lys Ala Ile Ala Asp Gln Ala Ala Arg Leu Ile Gln Tyr

65                  70                  75                  80

Thr Pro Ala Tyr Phe Ala Asn Ser Gln Ala Ala Arg Leu Ala Pro Arg
                85                  90                  95

Leu Ala Ala Leu Ala Pro Ile Ser Gly Pro Val Glu Val Ala Trp Gly
            100                 105                 110

Asn Ser Gly Ser Asp Ala Asn Asp Ala Ile Ile Lys Phe Ala Arg Ala
        115                 120                 125

Ala Thr Gly Arg Gln Tyr Ile Val Ser Phe Thr Gly Ala Tyr His Gly
    130                 135                 140

Ser Thr Tyr Gly Ser Met Thr Ile Ser Ser Val Thr Leu Asn Met Ser
145                 150                 155                 160

Arg Lys Met Gly Pro Leu Leu Pro Gly Val Val Lys Val Pro Phe Pro
                165                 170                 175

Ser Pro Trp Asp Lys Leu Ala Gly Glu Ser Asp Asp Gln Phe Val Asp
            180                 185                 190

Arg Met Tyr Lys Ala Phe Leu Gln Pro Phe Glu Thr Tyr Leu Pro Ala
        195                 200                 205

Glu Glu Val Ala Ala Ile Val Ile Glu Pro Ile Gln Gly Asp Gly Gly
    210                 215                 220

Ile Ile Lys Val Pro Gln Ala Tyr Met Glu Arg Ile Tyr Ala Phe Ala
225                 230                 235                 240

Lys Gln Asn Gly Ile Leu Phe Ala Ile Asp Glu Val Asn Gln Gly Met
                245                 250                 255

Gly Arg Thr Gly Lys Trp Trp Ser Ile Gln His Phe Pro Gly Ile Glu
            260                 265                 270

Pro Asp Leu Met Ser Val Gly Lys Ser Leu Ala Ser Gly Met Pro Leu
        275                 280                 285

Ser Ala Val Val Gly Arg Lys Gly Ile Met Glu Ala Leu Gly Pro Ala
    290                 295                 300

Ala Asn Val Tyr Thr Thr Ala Gly Asn Pro Val Thr Ala Ala Ala Ala
305                 310                 315                 320

Asn Ala Thr Leu Asp Val Ile Glu Glu Glu His Leu Val Glu Arg Ser
                325                 330                 335

Ala Lys Leu Gly Gln Lys Ala Lys Asp Phe Phe Asp Gln Glu Gln Ala
            340                 345                 350

Lys Tyr Asp Phe Val Gly Asp Ala Arg Leu Tyr Gly Leu Asn Gly Gly
        355                 360                 365

Ile Ser Ile Val Asp Pro Ala Thr Gly Lys Gly Asp Val Glu Ala Thr
    370                 375                 380

Thr Lys Leu Met Tyr Arg Ile Phe Glu Leu Gly Ala Ile Ile Ile Ser
385                 390                 395                 400

Leu Arg Gly Asn Ile Leu Arg Phe Gln Pro Pro Leu Val Ile Thr Glu
                405                 410                 415

Glu Gln Leu Asp Lys Ala Phe Ala Ile Ile Asp Gln Ala Phe Ala Glu
            420                 425                 430

Leu Ala Ala Gly Lys Leu Ser Leu Pro Ser Asn Ala Glu Glu Leu Gly
        435                 440                 445

Trp Glu
    450


<210>  4
<211>  456
<212>  PRT
<213>  Lactobacillus reuteri DSM 20016

<400>  4

Met Asp Lys Met Leu Lys Thr Lys Leu Lys Gln Lys Lys Thr Asn Arg
1               5                   10                  15

Asp Val Leu Leu Glu Glu Lys Asn Ala Phe Ala Ser Ala Ala Arg Ile
            20                  25                  30

Lys Tyr Tyr Asp Ile Val Leu Asp His Gly Arg Gly Ala Met Val Thr
        35                  40                  45

Asp Ile Glu Gly Asn Asp Tyr Ile Asp Leu Leu Ala Ser Ala Ser Ser
    50                  55                  60

Thr Asn Thr Gly His Ala His Pro Arg Val Val Gln Ala Ile Gln Glu
65                  70                  75                  80

Gln Ala Ala Lys Met Ile Gln Tyr Thr Pro Ala Tyr Phe Ala Asn Ser
                85                  90                  95

Gln Ala Ala Arg Leu Ala Pro Arg Leu Ala Glu Leu Ala Pro Ile Ser
            100                 105                 110

Gly Pro Val Glu Met Val Trp Gly Asn Ser Gly Ser Asp Ala Asn Asp
        115                 120                 125

Ala Ile Ile Lys Phe Ala Arg Ala Tyr Thr Gly Arg Gln Tyr Ile Ile
    130                 135                 140

Ser Phe Thr Gly Ala Tyr His Gly Ser Thr Tyr Gly Ser Met Ser Leu
145                 150                 155                 160

Ser Gly Val Ser Leu Asn Met Thr Arg Lys Met Gly Pro Met Leu Pro
                165                 170                 175

Gly Val Val Lys Val Pro Phe Pro Ser Pro Trp Glu Arg Leu Thr Asn
            180                 185                 190

Glu Ser Asp Glu Ala Phe Val Glu Arg Met Phe His Gln Phe Met Leu
        195                 200                 205

Pro Phe Glu Thr Tyr Leu Pro Val Asp Glu Val Ala Ala Ile Leu Ile
    210                 215                 220

Glu Pro Ile Gln Gly Asp Gly Gly Ile Val Lys Thr Pro Pro Ala Tyr
225                 230                 235                 240

Met Gln Lys Val Tyr Glu Phe Ala Lys Ala Asn Gly Ile Leu Phe Ala
                245                 250                 255

Val Asp Glu Val Asn Gln Gly Met Gly Arg Thr Gly Lys Trp Trp Ser
            260                 265                 270

Ile Gln His Phe Gly Ile Glu Pro Asp Leu Met Ser Val Gly Lys Ser
        275                 280                 285

Leu Ala Ser Gly Met Pro Leu Ser Ala Met Ile Gly Arg Lys Glu Ile
    290                 295                 300

Leu Glu Ser Leu Gly Ala Pro Ala Asn Val Tyr Thr Thr Ala Gly Asn
305                 310                 315                 320

Pro Val Thr Thr Ala Ala Ala Asn Ala Thr Leu Asp Val Ile Gln Asn
                325                 330                 335

Glu His Leu Leu Glu Arg Ser Gln Arg Leu Gly Lys Lys Ala Ala Glu
            340                 345                 350

Phe Phe Lys Leu Glu Gln Gln Lys Tyr Pro Phe Val Gly Asp Val Arg
        355                 360                 365

Met Tyr Gly Leu Asp Gly Gly Ile Asp Ile Ile Asp Pro Val Thr Gly
    370                 375                 380

Lys Gly Asp Thr Glu Ala Thr Thr Lys Leu Met Tyr Arg Ile Phe Glu
385                 390                 395                 400

Leu Gly Ala Ile Ile Ile Ser Leu Arg Gly Ser Ile Leu Arg Phe Gln
                405                 410                 415

Pro Pro Leu Val Ile Thr Glu Lys Glu Leu Asp Gln Ala Phe Ala Met
            420                 425                 430

Ile Asp Gln Ala Phe Ala Glu Leu Ala Ala Gly Lys Leu Ala Ala Pro
        435                 440                 445

Ala Asn Ala Glu Glu Leu Gly Trp
    450                 455

<210>  5
<211>  445
<212>  PRT
<213>  Staphyrococcus epidermidis ATCC 12228

<400>  5

Met Ser Lys Val Asn His Leu Ile Asp Glu Asp Glu Arg Tyr Phe Ala
1               5                   10                  15

His Ser Gly Arg Ile Lys Tyr Tyr Pro Leu Val Ile Asp His Gly Tyr
            20                  25                  30

Gly Ala Thr Leu Ile Asp Ile Asp Gly Lys Ser Tyr Ile Asp Leu Leu
        35                  40                  45

Ala Ser Ala Ser Ser Gln Asn Val Gly His Ala Pro Lys Pro Val Val
    50                  55                  60

Glu Ala Ile Lys Asn Gln Thr Glu Lys Phe Ile His Tyr Thr Pro Ala
65                  70                  75                  80

Tyr Met Tyr His Glu Pro Leu Val Arg Leu Ser Lys Lys Leu Cys Asp
                85                  90                  95

Ile Ala Pro Gly Asn Tyr Glu Lys Arg Val Thr Phe Gly Leu Ser Gly
            100                 105                 110

Ser Asp Ala Asn Asp Gly Ile Ile Lys Phe Ala Arg Ala Tyr Thr Gly
        115                 120                 125

Arg Pro Tyr Ile Ile Ser Phe Thr Asn Ala Tyr His Gly Ser Thr Phe
    130                 135                 140

Gly Ser Leu Ser Met Ser Ser Ile Ser Leu Asn Met Arg Lys Arg Tyr
145                 150                 155                 160

Gly Pro Leu Leu Asn Gly Phe Tyr His Ile Pro Phe Pro Asp Lys Tyr
                165                 170                 175

Arg Gly Met Phe Glu Gln Ala Lys Pro Asn Thr Val Glu Glu Tyr Leu
            180                 185                 190

Ala Pro Leu Lys Glu Met Phe Thr Lys Tyr Val Pro Ala Glu Glu Val
        195                 200                 205

Ala Cys Ile Val Val Glu Thr Ile Gln Gly Asp Gly Gly Leu Leu Glu
    210                 215                 220

Pro Val Pro Gly Tyr Phe Glu Ala Leu Gln Glu Leu Cys His Ala His
225                 230                 235                 240

Asn Ile Leu Ile Ala Val Asp Asp Ile Gln Gln Gly Leu Gly Arg Thr
                245                 250                 255

Gly Lys Trp Ser Ser Val Asp His Tyr His Phe Thr Pro Asp Leu Met
            260                 265                 270

Thr Phe Gly Lys Ser Leu Ala Gly Gly Leu Pro Met Ser Ala Ile Val
        275                 280                 285

Gly Arg Lys Glu Ile Met Glu Ser Leu Glu Ala Pro Ala His Leu Phe
    290                 295                 300

Thr Thr Gly Ala Asn Pro Val Ser Cys Glu Ala Ala Leu Ala Thr Ile
305                 310                 315                 320

Lys Met Ile Glu Asp Glu Asp Leu Leu Asn Ala Ser Trp Lys Lys Gly
                325                 330                 335

Ser Tyr Val Arg Lys Arg Ile Asp Pro Trp Ile Glu Arg Tyr Gln Tyr
            340                 345                 350

Val Gly Asp Val Arg Gly Ile Gly Leu Ser Ile Gly Ile Asp Ile Val
        355                 360                 365

Ser Asn Lys Ile Glu Lys Thr Arg Asp Ser Glu Ala Ala Leu Lys Ile
    370                 375                 380

Cys Asn Tyr Cys Phe Glu Asn Gly Val Ile Ile Ile Ala Val Ala Gly
385                 390                 395                 400

Asn Val Leu Arg Phe Gln Pro Pro Leu Val Ile Thr Tyr Lys Gln Leu
                405                 410                 415

Asp Lys Ala Leu Asp Thr Ile Glu Glu Ala Leu Glu Lys Leu Glu Arg
            420                 425                 430

Gly Glu Leu Asn Gln Tyr Asp Ile Ser Gly Gln Gly Trp
        435                 440                 445

<210>  6
<211>  444
<212>  PRT
<213>  Leuconostoc citreum NBRC 102476

<400>  6

Met Ser Asn Asn Lys Lys Ile Leu Ala Glu Glu Lys Arg Tyr Leu Ala
1               5                   10                  15

Asp Thr Ser Arg Val Lys Tyr Phe Asp Val Val Ile Asp His Gly Lys
            20                  25                  30

Gly Ala Thr Ile Thr Asp Ala Asp Gly Arg Asp Tyr Ile Asp Leu Leu
        35                  40                  45

Ala Ser Ala Ser Ala Thr Asn Thr Gly His Ser His Pro His Val Val
    50                  55                  60

Ala Ala Ile Ala Glu Gln Ala Glu Lys Leu Ile Gln Tyr Thr Pro Ala
65                  70                  75                  80

Tyr Phe Ala Asn Thr Thr Thr Ala Lys Leu Thr Gln Arg Leu Ala Lys
                85                  90                  95

Leu Ala Pro Gly Gln Phe Glu Lys Gln Val Val Phe Gly Asn Ser Gly
            100                 105                 110

Ser Asp Ala Asn Asp Ala Ile Ile Lys Phe Ala Arg Ala Tyr Thr Gly
        115                 120                 125

Arg Gln Tyr Ile Val Ser Phe Thr Gly Ala Tyr His Gly Ser Thr Tyr
    130                 135                 140

Gly Ala Ile Ser Ile Ser Gly Val Ser Leu Asn Met Thr Arg Lys Ile
145                 150                 155                 160

Gly Pro Leu Leu Pro Glu Val Val Lys Val Pro Phe Pro Asp Gln Asn
                165                 170                 175

Gln Arg Leu Ala Asn Glu Ser Asp Asp Asp Phe Ser Glu Arg Leu Phe
            180                 185                 190

Lys Gln Phe Lys Leu Pro Phe Glu Thr Tyr Leu Pro Ala Asp Asp Val
        195                 200                 205

Ala Leu Val Ile Ile Glu Pro Ile Gln Gly Asp Gly Gly Ile Val Lys
    210                 215                 220

Ala Pro Gln Lys Tyr Met Asp Leu Val Tyr Gln Phe Thr Arg Asp His
225                 230                 235                 240

Gly Ile Val Phe Ala Val Asp Glu Val Asn Gln Gly Leu Gly Arg Thr
                245                 250                 255

Gly Lys Leu Trp Ser Ile Asp His Phe Gly Ile Ala Pro Asp Leu Met
            260                 265                 270

Ser Val Gly Lys Ser Ile Ala Ser Gly Leu Pro Leu Ser Ala Val Ile
        275                 280                 285

Gly Arg Arg Glu Ile Met Ala Ser Leu Ser Ala Pro Ala Asn Val Tyr
    290                 295                 300

Thr Thr Ala Gly Asn Pro Val Thr Ala Ala Ala Ala Met Ala Thr Leu
305                 310                 315                 320

Asp Val Ile Glu Thr Glu Lys Leu Val Ala Arg Ser Glu Arg Ile Gly
                325                 330                 335

Asn Phe Ala Lys Ala Phe Phe Glu Lys Ala Ala Lys Arg Tyr Asp Phe
                340                 345                 350

Ile Gly Asp Val Arg Met Tyr Gly Leu Asn Gly Gly Ile Asp Ile Val
        355                 360                 365

Asp Ser Gln Gly Gln Pro Asp Asp Glu Ala Thr Thr Lys Leu Ile Tyr
    370                 375                 380

Arg Ile Phe Glu Leu Gly Ala Ile Met Ile Ser Leu Arg Gly Asn Thr
385                 390                 395                 400

Leu Arg Phe Gln Pro Pro Leu Val Ile Thr Gln Ala Ala Leu Glu Ser
                405                 410                 415

Ala Phe Ala Met Ile Ser Gln Ala Phe Ser Glu Leu Glu Ala Gly Gln
                420                 425                 430

Leu Thr Leu Pro Glu Thr Asp Asn His Ile Gly Trp
        435                 440

<210>  7
<211>  466
<212>  PRT
<213>  Pyrococcus horikoshii OT-3

<400>  7

Met Val Ser Met Thr Lys Trp Asp Glu Ile Arg Lys Tyr Thr Ser Lys
1               5                   10                  15

Lys Ile Glu Lys Asn Leu Glu Ile Val Lys Leu Asp Glu Lys Tyr Ile
            20                  25                  30

Pro Arg Ala Ser Gly Phe Lys Tyr Tyr Pro Met Val Ile Glu Arg Ser
        35                  40                  45

Ser Gly Ser Arg Ile Trp Asp Lys Asp Gly Asn Glu Tyr Ile Asp Phe
    50                  55                  60

Leu Thr Ser Ala Ala Val Phe Asn Val Gly His Thr His Pro Gly Val
65                  70                  75                  80

Val Lys Ala Val Glu Glu Gln Ile Lys Lys Phe Phe Asn Tyr Thr Met
                85                  90                  95

Gly Tyr Leu Tyr Val Glu Pro Pro Val Arg Leu Ala Glu Leu Leu Val
            100                 105                 110

Glu Ile Thr Pro Gly Asn Phe Glu Lys Lys Val Thr Tyr Gly Phe Ser
        115                 120                 125

Gly Ser Asp Ala Val Asp Ser Ser Ile Lys Ala Ala Arg Ala Tyr Thr
    130                 135                 140

Lys Arg Val Asn Ile Ile Ser Phe Leu His Ser Tyr His Gly Met Thr
145                 150                 155                 160

Tyr Gly Ala Leu Ser Ala Thr Gly Ile Leu Asp Pro Lys Leu Lys Lys
                165                 170                 175

Leu Leu His Pro Met Gly Asn Phe His His Val Glu Phe Pro Asp Pro
            180                 185                 190

Tyr Arg Asn Ser Trp Gly Ile Asp Gly Tyr Glu Asp Pro Ser Glu Leu
        195                 200                 205

Ala Asn Arg Ala Leu Asp Glu Ile Glu Arg Lys Ile Lys Glu Leu Asn
    210                 215                 220

Glu Asp Val Ala Gly Ile Ile Ile Glu Pro Ile Gln Gly Asp Ala Gly
225                 230                 235                 240

Val Val Ile Pro Pro Glu Glu Phe Val Arg Asp Leu Lys Lys Leu Thr
                245                 250                 255

Glu Glu Tyr Gly Ile Val Phe Ile Asp Glu Glu Val Gln Thr Gly Met
            260                 265                 270

Gly Arg Thr Gly Arg Trp Trp Gly Ile Glu His Phe Gly Val Thr Pro
        275                 280                 285

Asp Leu Ile Val Ser Ala Lys Ala Leu Gly Gly Gly Met Pro Ile Ser
    290                 295                 300

Ala Val Val Gly Lys Ala Glu Ile Met Asp Ser Val Pro Val Pro Phe
305                 310                 315                 320

Phe Val Phe Thr His Ile Gly His Ala Val Asn Ala Ser Ala Ala Ile
                325                 330                 335

Ala Thr Ile Asn Val Ile Lys Glu Glu Lys Leu Val Glu Arg Ser Glu
            340                 345                 350

Lys Leu Gly Glu Tyr Met Leu Lys Arg Leu Arg Glu Leu Gln Glu Thr
        355                 360                 365

Tyr Pro Ile Ile Gly Asp Val Arg Gly Lys Gly Leu Leu Ile Gly Val
    370                 375                 380

Asp Ile Val Lys Glu Gly Thr Arg Glu Pro Asp Arg Ser Leu Ala Gln
385                 390                 395                 400

Lys Ile Ser Trp Arg Ala Trp Glu Lys Gly Leu Ile Met Ile Thr Phe
                405                 410                 415

Gly Lys His Gly Asn Val Leu Arg Ile Ala Pro Pro Leu Asn Ile Pro
            420                 425                 430

Gln Glu Asp Leu Asp Lys Gly Val Glu Ile Ile Glu Glu Ser Ile Lys
        435                 440                 445

Asp Ala Val Glu Gly Lys Ile Pro Asp Glu Val Leu Lys Phe Leu Arg
    450                 455                 460
Ala Trp
465


<210>  8
<211>  474
<212>  PRT
<213>  Pyrococcus horikoshii OT-3

<400> 8

Met Pro Phe Leu Pro Phe Ser Tyr Tyr Pro Phe Ser Leu Glu Val Ile
1               5                   10                  15

Leu Met Glu Tyr Pro Lys Ile Val Val Lys Pro Pro Gly Pro Arg Ala
            20                  25                  30

Lys Glu Leu Ile Glu Arg Glu Lys Lys Val Leu Ser Thr Gly Ile Gly
        35                  40                  45

Val Lys Leu Phe Pro Leu Val Pro Lys Arg Gly Phe Gly Pro Phe Ile
    50                  55                  60

Glu Asp Val Asp Gly Asn Val Phe Ile Asp Phe Leu Ala Gly Ala Ala
65                  70                  75                  80

Ala Ala Ser Thr Gly Tyr Ala His Pro Lys Leu Val Lys Ala Val Lys
                85                  90                  95

Glu Gln Val Glu Leu Ile Gln His Ser Met Ile Gly Tyr Thr His Ser
            100                 105                 110

Glu Arg Ala Ile Arg Val Ala Glu Lys Leu Val Glu Ile Ser Pro Ile
        115                 120                 125

Glu Asn Ser Lys Val Ile Phe Gly Leu Ser Gly Ser Asp Ala Val Asp
    130                 135                 140

Met Ala Ile Lys Val Ser Lys Phe Ser Thr Arg Arg Pro Trp Ile Leu
145                 150                 155                 160

Ala Phe Ile Gly Ala Tyr His Gly Gln Thr Leu Gly Ala Thr Ser Val
                165                 170                 175

Ala Ser Phe Gln Val Ser Gln Lys Arg Gly Tyr Ser Pro Leu Met Pro
            180                 185                 190

Asn Val Phe Trp Ile Pro Tyr Pro Asn Pro Phe Arg Asn Ile Trp Gly
        195                 200                 205

Ile Asn Gly Tyr Glu Glu Pro Asp Glu Leu Ile Asn Arg Val Leu Asp
    210                 215                 220

Tyr Leu Glu Tyr Tyr Val Phe Ser His Val Val Pro Pro Asp Glu Val
225                 230                 235                 240

Ala Ala Leu Phe Ala Glu Pro Ile Gln Gly Asp Ala Gly Ile Val Val
                245                 250                 255

Pro Pro Glu Asn Phe Phe Lys Glu Leu Lys Lys Leu Leu Glu Glu Tyr
            260                 265                 270

Gly Ile Leu Leu Val Met Asp Glu Val Gln Thr Gly Ile Gly Arg Thr
        275                 280                 285

Gly Lys Trp Phe Ala Ser Glu Trp Phe Asn Val Lys Pro Asp Met Ile
    290                 295                 300

Ile Phe Gly Lys Gly Val Ala Ser Gly Met Gly Leu Ser Gly Val Ile
305                 310                 315                 320

Gly Arg Lys Glu Ile Met Asp Ile Thr Ser Gly Ser Ala Leu Leu Thr
                325                 330                 335

Pro Ala Ala Asn Pro Val Ile Ser Ala Ala Ala Glu Ala Thr Leu Glu
            340                 345                 350

Ile Ile Glu Glu Glu Asn Leu Leu Lys Asn Ala Leu Glu Val Gly Glu
        355                 360                 365

Phe Ile Met Gly Arg Leu Lys Glu Ile Lys Glu Arg Phe Glu Ile Ile
    370                 375                 380

Gly Asp Val Arg Gly Lys Gly Leu Met Ile Gly Val Glu Ile Val Lys
385                 390                 395                 400

Glu Asn Gly Arg Pro Asp Pro Glu Met Thr Gly Lys Ile Cys Trp Arg
                405                 410                 415

Ala Phe Glu Leu Gly Leu Ile Leu Pro Ser Tyr Gly Met Phe Gly Asn
            420                 425                 430

Val Ile Arg Ile Thr Pro Pro Leu Val Leu Thr Lys Glu Val Ala Glu

435                 440                 445

Lys Ala Leu Glu Ile Ile Glu Arg Ala Ile Lys Asp Thr Leu Thr Gly
    450                 455                 460

Lys Val Glu Arg Lys Val Val Thr Trp His
465                 470

<210>  9
<211>  58
<212>  DNA
<213>  Artificial Sequence

<220>
<223>  primer

<400>  9
ctaaaaaagc ttgtaggagg tgtagaaaat gtcaaaaaat aaagagattt tagaagaa          58

<210>  10
<211>  22
<212>  DNA
<213>  Artificial Sequence

<220>
<223>  primer

<400>  10
cgtatgtcta gaattcctaa tc                                                 22

<210>  11
<211>  57
<212>  DNA
<213>  Artificial Sequence

<220>
<223>  primer

<400>  11
attttaagct tttaggaggc atagattatg aacaaatcag agcaacttat aaatgag           57

<210>  12
<211>  24
<212>  DNA
<213>  Artificial Sequence

<220>
<223>  primer

<400>  12
gtttatttaa attctagaag gttg                                               24

<210>  13
<211>  35
<212>  DNA
<213>  Artificial Sequence

<220>
<223>  primer

<400>  13
gagacactgc catatggagg agaaaaaaag taacc                                   35

<210>  14
<211>  33
<212>  DNA
<213>  Artificial Sequence

<220>
<223>  primer

<400>  14
ctgaattctt attcccaacc cagctcttct gcg                                    33


<210>  15
<211>  35
<212>  DNA
<213>  Artificial Sequence

<220>
<223>  primer

<400>  15
gaagatggac catatgttaa aaacgaaact gaagc                                  35


<210>  16
<211>  37
<212>  DNA
<213>  Artificial Sequence

<220>
<223>  primer

<400>  16
gattggtcga cttaccaacc taactcttct gcatttg                                37


<210>  17
<211>  32
<212>  DNA
<213>  Artificial Sequence

<220>
<223>  primer

<400>  17
gagggaacca tatgagtaag gttaatcatt tg                                     32


<210>  18
<211>  32
<212>  DNA
<213>  Artificial Sequence

<220>
<223>  primer

<400>  18
cagaattctt accaaccttg accactaatg tc                                     32


<210>  19
<211>  38
<212>  DNA
<213>  Artificial Sequence

<220>
<223>  primer

<400>  19

ggggcgggac atatgtctaa caataaaaaa attttagc 38

<210> 20
<211> 31
<212> DNA
<213> Artificial Sequence

<220>
<223> primer

<400> 20
gcgaattctt accagccaat gtgattatcc g 31

<210> 21
<211> 50
<212> DNA
<213> Artificial Sequence

<220>
<223> primer

<400> 21
gttctgctgc agggaggagg attaagagat ggtttccatg accaagtggg 50

<210> 22
<211> 32
<212> DNA
<213> Artificial Sequence

<220>
<223> primer

<400> 22
gagtgtgaat tcatggggat atgctagagt gt 32

<210> 23
<211> 29
<212> DNA
<213> Artificial Sequence

<220>
<223> primer

<400> 23
gaatcccata tgccttttct acccttttc 29

<210> 24
<211> 32
<212> DNA
<213> Artificial Sequence

<220>
<223> primer

<400> 24
gaagccgaat tcgagcagaa gcctgtgata cc 32

<210> 25
<211> 30
<212> DNA
<213> Artificial Sequence

<220>
<223> primer

<400>  125
cacaggaaac agcatatggc catgattacg                                        30


<210>  26
<211>  30
<212>  DNA
<213>  Artificial Sequence

<220>
<223>  primer

<400>  26
cgtaatcatg gccatatgct gtttcctgtg                                        30

## Description

### CROSS-REFERENCE TO RELATED APPLICATION

This application is based upon and claims the benefit of priority from the prior Japanese Patent Application No. 2009-154287, filed on June 29, 2009, the entire contents of which are incorporated herein by reference.

### BACKGROUND OF THE INVENTION

### Field of the Invention

The present invention relates to the method for racemization of optically active a(alpha)-amino acids.

### Related Art

In the course of manufacturing optically active D- or L-α-amino acids utilized as the intermediate for manufacturing a variety of industrial agents, agricultural agents and medicines by using an optical resolution agent, the racemization of the other remaining optically active D- or L-α-amino acid into a racemic α-amino acid as a raw material again is an important method.

As a method for racemizing an optically active α-amino acid, chemical racemization methods are known such as the methods for racemizing an α-amino acid at high temperature in an acidic or alkaline aqueous solution (US Patent Nos. 2586154 and 4769486). However, these methods use severe reaction conditions resulting in many by-products. Racemization methods with catalysts are also known such as the methods for racemization with salicylaldehyde as an catalyst under the acidic conditions (Japanese Patent Laid-Open Publication H11-228512 and H11-322684 (European Patent corresponding to the previous two Japanese Patent Laid-Open Publications being EP 0937705A)). However, these methods also have severe reaction conditions thus giving the α-amino acid with decreased yields.

The racemization methods with enzymes are also known as the method for racemizing an optically active α-amino acid. In general, enzymes such as alanine racemase, glutamate racemase and proline racemase racemize amino acids. However, these enzymes generally have a high substrate specificity and are unsuited for manufacturing the racemic α-amino acid from a variety of optically active α-amino acids.

As the enzymes for racemizing optically active α-amino acids, amino acid racemases having low substrate specificity are also known (International Publication No. 2003/074690, (Kenji Soda, Sei-kagaku Jikken Koza 11: Metabolism of amino acids, and biogenic amines (I), Tokyo Kagaku Dojin, P. 275-296 (1976))). However, said enzymes exhibits only the low activity to optically active aliphatic α-amino acids and are hardly applied to the racemization of a variety of optically active α-amino acid.

As the other methods, there is also known the method for racemization of α-amino acids with microorganisms (Japanese Patent Laid-Open Publication No. 2004-57014). However, the microorganisms disclosed herein have only racemization activity to aromatic α-amino acids and thus are hardly applied to the racemization of a variety of optically active α-amino acid as described above.

While several methods for racemizing the optically active α-amino acids are known as described above, all of these methods have some problems. Therefore, an efficient racemization method which can be applied to a variety of optically active α-amino acids has still been demanded.

### SUMMARY OF THE INVENTION

The present inventors have now unexpectedly found that an enzyme classified to γ(gamma)-aminobutyric acid aminotransferase which is originally involved in the reaction of γ-aminobutyric acid and 2-oxoglutaric acid has an activity for racemizing a wide variety of optically active α-amino acids. The present invention is based on such findings.

The object of the present invention is to provide a method for inexpensively manufacturing a racemic α-amino acid of high quality in the course of manufacturing an optically active D- or L-α-amino acid utilized as the intermediate for manufacturing a variety of industrial agents, agricultural agents and medicines by using an optical resolution agent which may be further used as a raw material from the other remaining optically active D- or L-α-amino acid. It will be possible to further obtain the desired optically active α-amino acid in a high yield from the racemic α-amino acid thus obtained as the raw material.

That is, the following inventions are provided according to the present invention:
(1) A method for racemization of an optically active α-amino acid, comprising racemizing an optically active α-amino acid with γ-aminobutyric acid aminotransferase producing viable bacterial cells derived from genera *Leuconostoc, Lactobacillus, Weissella, Oenococcus, Staphylococcus, Aminobacterium, Alkaliphilus, Clostridium, Pyrococcus, Thermococcus, Thermofilum, Pyrobaculum, Anoxybacillus, Syntrophus, Thermoproteus, Symbiobacterium, Desulfurococcus, Bacillus, Acidobacteria, Clostridium, Deinococcus, Pelobacter, Geobacillus, Geobacter, Pelotomaculum, Solibacter, Coprothermobacter, Aeropyrum, Gloeobacter, Nostoc, Herpetosiphon, Metallosphaera, Syntrophobacter, Photorhabdus, Caldivirga, Hyperthermus, Thermoplasma, Gemmata, Anabaena, Cyanothece, Picrophilus* or *Chloroflexus,* or a processed product thereof.

(2) A method according to (1), wherein γ-aminobutyric acid aminotransferase is derived from bacteria belonging to:
*Leuconostoc mesenteroides subsp. mesenteroides,*
*Staphylococcus saprophyticus subsp. saprophyticus,*
*Lactobacillus fermentum,*
*Lactobacillus reuteri,*
*Staphylococcus epidermidis,*
*Leuconostoc citreum,* or
*Pyrococcus horikoshii.*

(3) A method according to (1) or (2), wherein γ-aminobutyric acid aminotransferase is derived from:
*Leuconostoc mesenteroides subsp. mesenteroides* ATCC 8293,
*Staphylococcus saprophyticus subsp, saprophyticus* ATCC 15305,
*Lactobacillus fermentum* IFO 3956,
*Lactobacillus reuteri* DSM 20016,
*Staphylococcus epidermidis* ATCC 12228,
*Leuconostoc citreum* NBRC 102476, or
*Pyrococcus horikoshii* OT-3.

(4) A method according to any one of (1) to (3), wherein γ-aminobutyric acid aminotransferase is a polypeptide selected from:
(a) a polypeptide consisting of an amino acid sequence selected from the group consisting of the amino acid sequences of SEQ ID NOS: 1, 2, 3, 4, 5, 6, 7 and 8,
(b) a polypeptide consisting of the amino acid sequence in which one or more amino acid residues are deleted, substituted or added in said amino acid sequence of (a), or
(c) a polypeptide consisting of the amino acid sequence having 75% or higher identity to said amino acid sequence of (a).

(5) A method for racemization of optically active α-amino acids, comprising racemizing optically active α-amino acids with γ-aminobutyric acid aminotransferase selected from:
(a) a polypeptide consisting of an amino acid sequence selected from the group consisting of the amino acid sequences of SEQ ID NOS: 1, 2, 3, 4, 5, 6, 7 and 8,
(b) a polypeptide consisting of the amino acid sequence in which one or more amino acid residues are deleted, substituted or added in said amino acid sequence of (a), or
(c) a polypeptide consisting of the amino acid sequence having 75% or higher identity to said amino acid sequence of (a).

(6) A method according to any one of (1) to (5), wherein γ-aminobutyric acid aminotransferase is a polypeptide derived from *Leuconostoc mesenteroides subsp. mesenteroides* ATCC 8293 and selected from:
(a1) a polypeptide consisting of an amino acid sequence selected from the group consisting of the amino acid sequence of SEQ ID NO: 1,
(b1) a polypeptide consisting of the amino acid sequence in which one or more amino acid residues are deleted, substituted or added in said amino acid sequence of (a1), or
(c1) a polypeptide consisting of the amino acid sequence having 75% or higher identity to said amino acid sequence of (a1).

(7) A method according to any one of (1) to (5), wherein γ-aminobutyric acid aminotransferase is a polypeptide derived from *Staphylococcus saprophyticus subsp. saprophyticus* ATCC 15305 and selected from:
(a2) a polypeptide consisting of an amino acid sequence selected from the group consisting of the amino acid sequence of SEQ ID NO: 2,
(b2) a polypeptide consisting of the amino acid sequence in which one or more amino acid residues are deleted, substituted or added in said amino acid sequence of (a2), or
(c2) a polypeptide consisting of the amino acid sequence having 75% or higher identity to said amino acid sequence of (a2).

(8) A method according to any one of (1) to (5), wherein γ-aminobutyric acid aminotransferase is a polypeptide derived from *Lactobacillus fermentum* IFO 3956 and selected from:
(a3) a polypeptide consisting of an amino acid sequence selected from the group consisting of the amino acid sequence of SEQ ID NO: 3,
(b3) a polypeptide consisting of the amino acid sequence in which one or more amino acid residues are deleted, substituted or added in said amino acid sequence of (a3), or
(c3) a polypeptide consisting of the amino acid sequence having 75% or higher identity to said amino acid sequence of (a3).

(9) A method according to any one of (1) to (5), wherein γ-aminobutyric acid aminotransferase is a polypeptide derived from *Lactobacillus reuteri* DSM 20016 and selected from:
(a4) a polypeptide consisting of an amino acid sequence selected from the group consisting of the amino acid sequence of SEQ ID NO: 4,
(b4) a polypeptide consisting of the amino acid sequence in which one or more amino acid residues are deleted, substituted or added in said amino acid sequence of (a4), or
(c4) a polypeptide consisting of the amino acid sequence having 75% or higher identity to said amino acid sequence of (a4).

(10) A method according to any one of (1) to (5), wherein γ-aminobutyric acid aminotransferase is a polypeptide derived from *Staphylococcus epidermidis* ATCC 12228 and selected from:
(a5) a polypeptide consisting of an amino acid sequence selected from the group consisting of the amino acid sequence of SEQ ID NO: 5,
(b5) a polypeptide consisting of the amino acid sequence in which one or more amino acid residues are deleted, substituted or added in said amino acid sequence of (a5), or
(c5) a polypeptide consisting of the amino acid sequence having 75% or higher identity to said amino acid sequence of (a5).

(11) A method according to any one of (1) to (5), wherein γ-aminobutyric acid aminotransferase is a polypeptide derived from *Leuconostoc citreum* NBRC 102476 and selected from:
(a6) a polypeptide consisting of an amino acid sequence selected from the group consisting of the amino acid sequence of SEQ ID NO: 6,
(b6) a polypeptide consisting of the amino acid sequence in which one or more amino acid residues are deleted, substituted or added in said amino acid sequence of (a6), or
(c6) a polypeptide consisting of the amino acid sequence having 75% or higher identity to said amino acid sequence of (a6).

(12) A method according to any one of (1) to (5), wherein γ-aminobutyric acid aminotransferase is a polypeptide derived from *Pyrococcus horikoshii* OT-3 and selected from:
(a7) a polypeptide consisting of an amino acid sequence selected from the group consisting of the amino acid sequences of SEQ ID NOS: 7 or 8,
(b7) a polypeptide consisting of the amino acid sequence in which one or more amino acid residues are deleted, substituted or added in said amino acid sequence of (a7), or
(c7) a polypeptide consisting of the amino acid sequence having 75% or higher identity to said amino acid sequence of (a7).

(13) A method according to any one of (1) to (12), wherein the optically active α-amino acid comprises one or more of the compounds represented by the following formula (I): 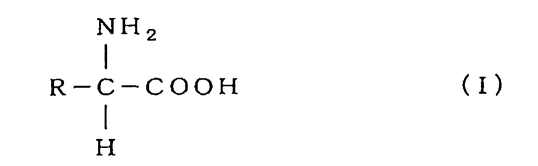 wherein
R represents
a benzyl group which is optionally substituted by methyl or hydroxy groups, or
a linear or branched alkyl group having 1 to 4 carbon atoms, wherein the alkyl group is optionally substituted by hydroxy, methylthio, thiol, amide, or imidazolyl groups.

(14) A method according to (13), wherein optically active α-amino acid comprises one or more of the compounds selected from the group consisting of alanine, valine, leucine, isoleucine, tert-leucine, histidine, phenylalanine, methionine, asparagine, tyrosine, serine and 2-aminobutyric acid.

(15) A method according to any one of (1) to (14), wherein the optically active α-amino acid is the L form.

(16) A method according to any one of (1) to (14), wherein the optically active α-amino acid is the D form.

Moreover, according to another embodiment of the present invention, the following inventions are provided:
(2') A method for racemization of optically active α-amino acids according to (1), wherein the γ-aminobutyric acid aminotransferase is an amino acid sequence derived from *Leuconostoc mesenteroides subsp. mesenteroides* ATCC 8293, represented by SEQ ID NO: 1, or an amino acid sequence in which one or more amino acid residues have been deleted, substituted or added from the amino acid sequence of SEQ ID NO: 1.
(3') A method for racemization of optically active α-amino acids according to (1), wherein the γ-aminobutyric acid aminotransferase is derived from *Staphylococcus saprophyticus subsp. saprophyticus* ATCC 15305, represented by SEQ ID NO: 2, or an amino acid sequence in which one or more amino acid residues have been deleted, substituted or added from the amino acid sequence of SEQ ID NO: 2.

(4') A method for racemization of optically active α-amino acids according to (1), wherein the γ-aminobutyric acid aminotransferase is derived from *Lactobacillus fermentum* IFO 3956, represented by SEQ ID NO: 3, or an amino acid sequence in which one or more amino acid residues have been deleted, substituted or added from the amino acid sequence of SEQ ID NO: 3.
(5') A method for racemization of optically active α-amino acids according to (1), wherein the γ-aminobutyric acid aminotransferase is derived from *Lactobacillus reuteri* DSM 20016, represented by SEQ ID NO: 4, or an amino acid sequence in which one or more amino acid residues have been deleted, substituted or added from the amino acid sequence of SEQ ID NO: 4.

(6') A method for racemization of optically active α-amino acids according to (1), wherein the γ-aminobutyric acid aminotransferase is derived from *Staphylococcus epidermidis* ATCC 12228, represented by SEQ ID NO: 5, or an amino acid sequence in which one or more amino acid residues have been deleted, substituted or added from the amino acid sequence of SEQ ID NO: 5.
(7') A method for racemization of optically active α-amino acids according to (1), wherein the γ-aminobutyric acid aminotransferase is derived from *Leuconostoc citreum* NBRC 102476, represented by SEQ ID NO: 6, or an amino acid sequence in which one or more amino acid residues have been deleted, substituted or added from the amino acid sequence of SEQ ID NO: 6.

(8') A method for racemization of optically active α-amino acids according to (1), wherein the γ-aminobutyric acid aminotransferase is derived from *Pyrococcus horikoshii* OT-3, represented by SEQ ID NOS: 7 or 8, or an amino acid sequence in which one or more amino acid residues have been deleted, substituted or added from the amino acid sequence of SEQ ID NOS: 7 or 8.
(9') A method for racemization of optically active α-amino acids according to (1), wherein the γ-aminobutyric acid aminotransferase producing bacterial cell or a processed product thereof is represented by SEQ ID NOS: 1, 2, 3, 4, 5, 6, 7 or 8, or a gene recombinant bacterium modified in such a manner that γ-aminobutyric acid aminotransferase which is an amino acid sequence in which one or more amino acid residues have been deleted, substituted or added from the amino acid sequence of SEQ ID NOS: 1, 2, 3, 4, 5, 6, 7 or 8 is produced, or a processed product thereof.

(10') A method for racemization of optically active α-amino acids according to any one of (1) to (9'), wherein the optically active α-amino acid is at least one selected from alanine, valine, leucine, phenylalanine, methionine, serine and 2-aminobutyric acid.

In the present invention, the racemization of an optically active α-amino acid can be performed without processes such as the derivatization of α-amino acids by using γ-aminobutyric acid aminotransferase producing viable bacterial cells which have the racemization activity of α-amino acids, or a processed product thereof. According to the present invention, a variety of optically active α-amino acids of high quality which are useful as the intermediate for manufacturing a variety of industrial agents, agricultural agents and medicines can be racemized efficiently and inexpensively. The other L- or D-α-amino acid hitherto remaining after the optical resolution of an racemic α-amino acid can be effectively used as the raw material to give the desired optically active amino acid and thus very useful in the fields of medicine, agricultural agents and the like.

### DETAILED DESCRIPTION OF THE INVENTION

### γ-Aminobutyric acid aminotransferase

The γ-aminobutyric acid aminotransferase used in the present invention has the racemization activity of optically active α-amino acids.

Herein, the phraseology "has the racemization activity of optically active α-amino acids" means to racemize an optically active α-amino acid, that is, the α-amino acid consisting of only one of the L-or D-isomer or the mixture of the L- and D-isomers of which the abundance ratio is biased to any one of the forms, and whether the intended enzyme (or polypeptide) has such racemization activity or not may be measured according to the conventional methods including those described later in examples (Examples 1 to 5), by which the activity can be confirmed. As regards the confirmation of having the racemization activity or not, further specifically the desired enzyme used according to the description of the examples can be judged active if the ratio of the L- and D-isomers is varied after the reaction with the L- or D-isomer of the α-amino acid as the substrate. The variation in this case is the one that the optical activity decreases. Even if the variation in the ratio of the L- and D-isomers is confirmed little, the variation range can be changed by changing the amounts of bacterial cells and enzyme.

In the present invention, the γ-aminobutyric acid aminotransferase is typically derived from genera *Leuconostoc, Lactobacillus, Weissella, Oenococcus, Staphylococcus, Aminobacterium, Alkaliphilus, Clostridium, Pyrococcus, Thermococcus, Thermofilum, Pyrobaculum, Anoxybacillus, Syntrophus, Thermoproteus, Symbiobacterium, Desulfurococcus, Bacillus, Acidobacteria, Clostridium, Deinococcus, Pelobacter, Geobacillus, Geobacter, Pelotomaculum, Solibacter, Coprothermobacter, Aeropyrum, Gloeobacter, Nostoc, Herpetosiphon, Metallosphaera, Syntrophobacter, Photorhabdus, Caldivirga, Hyperthermus, Thermoplasma, Gemmata, Anabaena, Cyanothece, Picrophilus* or *Chloroflexus.*

Preferably, said γ-aminobutyric acid aminotransferase is derived from genera *Leuconostoc, Lactobacillus, Weissella, Oenococcus, Staphylococcus, Aminobacterium, Alkaliphilus, Clostridium, Pyrococcus, Thermococcus* or *Thermofilum,* and more preferably, from genera *Leuconostoc, Lactobacillus, Oenococcus, Staphylococcus, Aminobacterium, Alkaliphilus, Clostridium* or *Pyrococcus.*

According to the further preferred embodiment of the present invention, the γ-aminobutyric acid aminotransferase is derived from:
*Leuconostoc mesenteroides subsp. mesenteroides,*
*Staphylococcus saprophyticus subsp. saprophyticus,*
*Lactobacillus fermentum,*
*Lactobacillus reuteri,*
*Staphylococcus epidermidis,*
*Leuconostoc citreum,* or
*Pyrococcus horikoshii,* and
more preferably
*Leuconostoc mesenteroides subsp. mesenteroides* ATCC 8293,
*Staphylococcus saprophyticus subsp. saprophyticus* ATCC 15305,
*Lactobacillus fermentum* IFO 3956,
*Lactobacillus reuteri* DSM 20016,
*Staphylococcus epidermidis* ATCC 12228,
*Leuconostoc citreum* NBRC 102476, or
*Pyrococcus horikoshii* OT-3.

The γ-aminobutyric acid aminotransferase in the present invention is a polypeptide selected from:
(a) a polypeptide composed of an amino acid sequence selected from the group consisting of the amino acid sequences of SEQ ID NOS: 1, 2, 3, 4, 5, 6, 7 and 8,
(b) a polypeptide consisting of the amino acid sequence in which one or more amino acid residues are deleted, substituted or added in said amino acid sequence of (a), or
(c) a polypeptide consisting of the amino acid sequence having 75% or higher identity to said amino acid sequence of (a).

The polypeptide (b) described above (referred to hereinafter as "modified polypeptide") comprises any one of the amino acid sequences selected from the group consisting of the amino acid sequences of SEQ ID NOS: 1, 2, 3, 4, 5, 6, 7 and 8 in which one or more amino acid residues have been deleted, substituted or added. In this context, the number of the amino acids which may be deleted, substituted or added is, for example, in the range of 1 to 40, preferably 1 to 30, more preferably 1 to 20, further preferably 1 to 10, particularly preferably 1 to 8, most preferably 1 to 4.

The term "deleted" (or lost) also includes polypeptides that amino acid residues have been lost from the terminal of an amino acid sequence and that amino acid residues have been deleted in the middle of an amino acid sequence.
Furthermore, the term "added" also includes polypeptides that amino acid residues have been added to the terminal of an amino acid sequence and that amino acid residues have been added (inserted) to the middle of an amino acid sequence.

The modified polypeptide described in (b) may be a polypeptide comprising any one of the amino acid sequences selected from the group consisting of the amino acid sequences of SEQ ID NOS: 1, 2, 3, 4, 5, 6, 7 and 8 in which one or more amino acid residues have been conservatively substituted and having the racemization activity of optically active α-amino acids.

In this context, the term "conservatively substituted" means the substitution of one or more (preferably several) amino acid residues by the other chemical analogous amino acid residues so that the activity of the peptide will not substantially be modified. For instance, such substitutions include the substitutions of a hydrophobic residue by the other hydrophobic residue or of a polar residue by the other polar residue having the same charge. Functionally similar amino acids which can perform such substitutions are well known in the art with every amino acid. By way of specific examples, nonpolar (hydrophobic) amino acids include alanine, valine, isoleucine, leucine, proline, tryptophan, phenylalanine, methionine, and the like. Polar (neutral) amino acids include glycine, serine, threonine, tyrosine, glutamine, asparagine, cysteine, and the like. (Basic) amino acids having a positive charge include arginine, histidine, lysine, and the like. In addition, (acidic) amino acids having a negative charge include aspartic acid, glutamic acid, and the like.

In this connection, the modification of the amino acid by deletion, substitution or addition can be performed, for example, by subjecting DNA coding for the modification to site-specific mutagenesis as the well-known technique (see, for example, Nucleic Acid Research, Vol. 10, No. 20, p. 6487-6500, 1982). The other methods include a method for treating genes with mutagens, a method for selectively cleaving genes, followed by removing, adding and/or substituting the selected nucleotides, which are linked together, and the like.

The polypeptide (c) described above (referred to hereinafter as homologous polypeptide) is not specifically restricted as far as it is composed of the amino acid sequence having 75% or higher identity to an amino acid sequence selected from the group consisting of the amino acid sequences of SEQ ID NOS: 1, 2, 3, 4, 5, 6, 7 and 8, that is, the amino acid sequence of γ-aminobutyric acid aminotransferase. The "identity" is preferably in the range of 80% or more, more preferably 85% or more, further preferably 90% or more, further more preferably 95% or more, particularly preferably 98% or more, and most preferably 99% or more. The homologous polypeptide in (c) has the activity for racemizing the optically active α-amino acids as well as the polypeptide in (a).

In this specification, all of the values of the "identity" may only be values calculated with the homology retrieval program well known to persons in the art and may be calculated, for example, with a default parameter in the homology algorithm BLAST (Basic local alignment search tool)of the National Center for Biotechnology Information (NCBI)(http://www.ncbi.nlm.nih.gov/BLAST/).

The γ-aminobutyric acid aminotransferase in the present invention is preferably any one of the following (I) to (VII):
(I) a polypeptide derived from *Leuconostoc mesenteroides subsp. mesenteroides* ATCC 8293 and selected from:
   (a1) a polypeptide consisting of an amino acid sequence selected from the group consisting of the amino acid sequence of SEQ ID NO: 1,
   (b1) a polypeptide consisting of the amino acid sequence in which one or more amino acid residues are deleted, substituted or added in said amino acid sequence of (a1), or
   (c1) a polypeptide consisting of the amino acid sequence having 75% or higher identity to said amino acid sequence of (a1);

(II) a polypeptide derived from *Staphylococcus saprophyticus subsp. saprophyticus* ATCC 15305 and selected from:
(a2) a polypeptide consisting of an amino acid sequence selected from the group consisting of the amino acid sequence of SEQ ID NO: 2,
(b2) a polypeptide consisting of the amino acid sequence in which one or more amino acid residues are deleted, substituted or added in said amino acid sequence of (a2), or
(c2) a polypeptide consisting of the amino acid sequence having 75% or higher identity to said amino acid sequence of (a2);

(III) a polypeptide derived from *Lactobacillus fermentum* IFO 3956 and selected from:
(a3) a polypeptide consisting of an amino acid sequence selected from the group consisting of the amino acid sequence of SEQ ID NO: 3,
(b3) a polypeptide consisting of the amino acid sequence in which one or more amino acid residues are deleted, substituted or added in said amino acid sequence of (a3), or
(c3) a polypeptide consisting of the amino acid sequence having 75% or higher identity to said amino acid sequence of (a3);

(IV) a polypeptide derived from *Lactobacillus reuteri* DSM 20016 and selected from:
(a4) a polypeptide consisting of an amino acid sequence selected from the group consisting of the amino acid sequence of SEQ ID NO: 4,
(b4) a polypeptide consisting of the amino acid sequence in which one or more amino acid residues are deleted, substituted or added in said amino acid sequence of (a4), or
(c4) a polypeptide consisting of the amino acid sequence having 75% or higher identity to said amino acid sequence of (a4); or

(V) a polypeptide derived from *Staphylococcus epidermidis* ATCC 12228 and selected from:
(a5) a polypeptide consisting of an amino acid sequence selected from the group consisting of the amino acid sequence of SEQ ID NO: 5,
(b5) a polypeptide consisting of the amino acid sequence in which one or more amino acid residues are deleted, substituted or added in said amino acid sequence of (a5), or
(c5) a polypeptide consisting of the amino acid sequence having 75% or higher identity to said amino acid sequence of (a5); or

(VI) a polypeptide derived from *Leuconostoc citreum* NBRC 102476 and selected from:
(a6) a polypeptide consisting of an amino acid sequence selected from the group consisting of the amino acid sequence of SEQ ID NO: 6,
(b6) a polypeptide consisting of the amino acid sequence in which one or more amino acid residues are deleted, substituted or added in said amino acid sequence of (a6), or
(c6) a polypeptide consisting of an amino acid sequence having 75% or higher identity to said amino acid sequence of (a6); or

(VII) a polypeptide derived from *Pyrococcus horikoshii* OT-3 and selected from:
(a7) a polypeptide consisting of an amino acid sequence selected from the group consisting of the amino acid sequence of SEQ ID NO: 7,
(b7) a polypeptide consisting of the amino acid sequence in which one or more amino acid residues are deleted, substituted or added in said amino acid sequence of (a7), or
(c7) a polypeptide consisting of the amino acid sequence having 75% or higher identity to said amino acid sequence of (a7).
In this connection, the polypeptides in (b1) to (b7) and (c1) to (c7) have the activity for racemizing the optically active α-amino acids, and the number of the amino acids which may be deleted, substituted or added and the percentage of the identity can be confirmed according to (b) and (c).

### Method for racemization of optically active α-amino acids

The method for racemization of optically active α-amino acids of the present invention comprises the racemization of an optically active α-amino acid with a viable bacterial cell producing the γ-aminobutyric acid aminotransferase described above or a processed product thereof.

The microorganisms used for the biochemical racemization of the optically active α-amino acid of the present invention may only be the microorganisms which produce γ-aminobutyric acid aminotransferase having the racemization activity of an α-amino acid. In addition, all of the strains including mutant strains derived from these microorganisms by an artificial mutation means or recombinant strains derived by the genetic technologies such as cell fusion or gene recombination may be used in the present invention provided that these strains have the ability described above. In the present invention, genetic recombinant bacteria which have been modified so that the γ-aminobutyric acid aminotransferase described above is produced can be used suitably.

More particularly, the microorganisms producing the γ-aminobutyric acid aminotransferase can be obtained by the gene recombination of DNA coding for the γ-aminobutyric acid aminotransferase according to the method described in Molecular Cloning, 3rd Ed. (ed. Sambrook J. and Russell D.W.), Cold Spring Harbor, New York, 2001).

That is, the recombinant DNA is prepared by inserting the desired structural gene downstream the promoter of an appropriate vector. A transformant producing the γ-aminobutyric acid aminotransferase in the present invention can be obtained by introducing the recombinant DNA into host cells adapted to the expression vector.

As the host cells, any cell which can express the aimed gene including bacteria, actinomycetes, yeast, animal cells, insect cells, plant cells, and the like can be used. Preferably, there are mentioned bacteria including colon bacillus such as Escherichia coli, actinomycetes such as Streptomyces, and the like.

As the recombinant DNA, there is used the one which can self-replicate or can be integrated into chromosome in the host cells and has a promoter at the position in which the DNA of the present invention can be transcribed.

When prokaryotes such as bacteria are used as the host cells, the recombinant DNA comprising a DNA which codes for the protein of the present invention is preferably a recombinant DNA which can self-replicate in the prokaryote and is composed of a promoter, a ribosome binding sequence, the DNA of the present invention and a transcription terminating sequence. Furthermore, a gene controlling the promoter may be contained in the recombinant DNA.

The expression vector which can be used includes, for example, pTA2 (Toyobo Co., Ltd.), Helix 1 (Roche Diagnostics), pKK233-2 (Amersham Pharmacia Biotech), pSE280 (Invitrogen), pGEMEX-1 (Promega), pQE-8 (Quiagen), pET24 (Novagen), pBluescript II SK(+), pBluescript II SK(-) (Stratagene), pUC19 [Gene, 33, 103 (1985)], pSTV28 (Takara Shuzo Co., Ltd.), pUC118 (Takara Shuzo Co., Ltd.), pIJ6902 [Mol. Microbiol., 58, 1276 (2005)], pSET152 [J. Mol. Microbiol., 4, 417 (2002)], and the like.

As the promoter, any promoter which can work in the host cells can be used without particular limitation, and includes, for example, E. coli or phage derived promoters such as the trp promoter (Ptrp), the lac promoter (Plac), the T7 promoter, the PL promoter, the PR promoter, the PSE promoter, and the like.

The prokaryote as the host cells includes microorganisms belonging to genera *Escherichia, Serratia, Bacillus, Brevibacterium, Corynebacterium, Microbacterium, Streptomyces* and the like.

As the specific example, the method for preparing the E. coli DH5α strain newly prepared by the present inventors and having the ability for expressing γ-aminobutyric acid aminotransferase represented by SEQ ID NO: 1 or 2 is described below.
*Leuconostoc mesenteroides subsp. mesenteroides* ATCC 8293 or *Staphylococcus saprophyticus subsp. saprophyticus* ATCC 15305 as the DNA donor microorganism is cultured with a medium which generally contains assimilable carbon sources, nitrogen sources, inorganic salts essential to each microorganism, nutrients, and the like. The microorganism was cultured preferably at pH in the range of 4 to 10 and at a temperature in the range of 20 to 50 °C. Culture is aerobically conducted for about 1 day to 1 week.
In this connection, the condition for culturing microorganism to be cultured (medium composition, pH of medium, culturing temperature, the amount of bacterial cells seeded on the medium, culturing period, and the like) can be appropriately determined on the basis of the literatures corresponding to microbial species and the information shown in the culture collections and depositories.

A primer is designed on the basis of the genomic DNA base sequence information of *Leuconostoc mesenteroides subsp. mesenteroides* ATCC 8293 or *Staphylococcus saprophyticus subsp. saprophyticus* ATCC 15305. DNA coding for γ-aminobutyric acid aminotransferase is amplified with the chromosomal DNA extracted from *Leuconostoc mesenteroides subsp. mesenteroides* ATCC 8293 or *Staphylococcus saprophyticus subsp. saprophyticus* ATCC 15305 as the template and bonded with the plasmid vector pUC19. The product is named pAT8 with respect to the former template and pAT15 with respect to the latter template. pAT8 or pAT15 was transformed into E. coli (DH5α), and the recombinant bacteria expressing γ-aminobutyric acid aminotransferase are named DH5α/pAT8 and DH5α/pAT15.

General operations required for the handling of E. coli as the host of the recombinants are the ones usually practiced in the art and can be carried out, for example, according to Molecular Cloning, 3rd Ed.. Moreover, all of the enzymes and reagents to be used may be commercial products, and the object can be completely accomplished according to the working conditions of the products unless otherwise stated. The extraction of whole DNA from the bacteria can be performed, for example, according to the method of Saito et al. (Biochim. Biophys. Acta., 72, 619 - 629, 1963).

The microorganism producing the γ-aminobutyric acid aminotransferase may be appropriately cultured under the culture condition corresponding to the bacteria used without particular limitations, since each bacterium has its own optimal growth condition. For instance, the recombinant bacteria, of which host is E. coli DH5α, generally have the appropriate culture condition of pH 7 and the temperature of 37 °C. The microorganism thus cultured is used for reactions as the viable bacterial cell or the processed product thereof or as the purified enzyme.

In this connection, the processed product includes, for example, a culture fluid (culture), a culture concentrate, a dry culture, bacterial cells obtained by centrifuging the culture (separated bacterial cells), dry bacterial cells, lyophilized bacterial cells, surfactant processed bacterial cells, bacterial cell debris such as ultrasonic wave processed bacterial cells and mechanically ground product of the bacterial cells), solvent processed bacterial cells, enzyme processed bacterial cells, a protein fractionated product of the bacterial cells, or an enzyme preparation obtained by extracting from the bacterial cells. Moreover, the bacterial cells or the enzymes may be used by fixation.

The method for purifying the desired polypeptide from the culture (expressed cells or supernatant of culture) includes, for example, salting out with ammonium sulfate, ion exchange column chromatography with ion exchange cellulose, molecular sieve column chromatography with molecular sieve gel, affinity column chromatography with protein A linked polysaccharide, dialysis or lyophilization. Furthermore, when a synthetic method is used, the polypeptide can be synthesized according to the conventional methods such as liquid phase or solid phase method and an automatic synthesizer may generally be used. Chemically modified products can be synthesized by the conventional methods.

The optically active α-amino acid subjected to the racemization reaction may be any one of the D- or L-isomer or the mixture of the Land D-isomers of which the abundance ratio is biased to any one of the forms and specifically includes alanine, glutamine, glutamic acid, valine, leucine, isoleucine, tert-leucine, norvaline, norleucine, proline, phenylalanine, tryptophan, methionine, serine, threonine, cysteine, asparagine, tyrosine, lysine, arginine, histidine, aspartic acid, 2-aminobutyric acid, ornithine, and the like. These amino acids may be a combination of the two or more.

The optically active α-amino acid subjected to the racemization reaction as the raw material can also be represented by the following formula (I): 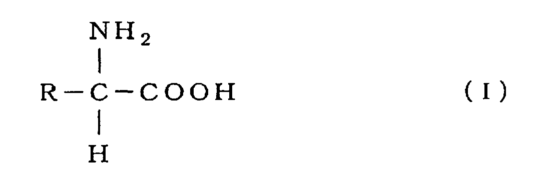 wherein
R represents
a benzyl group which is optionally substituted by methyl or hydroxy groups, or
a linear or branched alkyl group having 1 to 4 carbon atoms, wherein the alkyl group is optionally substituted by hydroxy (-OH), methylthio (-S-CH₃), thiol (-SH), amide (-C(=O)-NH₂), or imidazolyl groups.

The optically active α-amino acid subjected to the racemization reaction as the raw material is preferably selected from the group consisting of alanine, valine, leucine, isoleucine, norvaline, norleucine, tert-leucine, phenylalanine, tyrosine, methionine, serine, threonine, asparagine, glutamine, histidine, proline, 2-aminobutyric acid and ornithine, more preferably from the group consisting of alanine, valine, leucine, isoleucine, norvaline, norleucine, tert-leucine, phenylalanine, tyrosine, methionine, serine, asparagine, glutamine, histidine, 2-aminobutyric acid and ornithine, further preferably from the group consisting of alanine, valine, leucine, isoleucine, tert-leucine, asparagine, histidine, phenylalanine, methionine, asparagine, tyrosine, serine and 2-aminobutyric acid, further more preferably from the group consisting of alanine, valine, leucine, phenylalanine, methionine, serine and 2-aminobutyric acid. These amino acids may be the ones obtained either chemically or biologically and may contain impurities, but it is preferred to use a purified one.

Thus, according to another embodiment of the present invention, there is provided a method for racemization of optically active α-amino acids, comprising racemizing optically active α-amino acids with γ-aminobutyric acid aminotransferase selected from:
(a) a polypeptide composed of an amino acid sequence selected from the group consisting of the amino acid sequences of SEQ ID NOS: 1, 2, 3, 4, 5, 6, 7 and 8,
(b) a polypeptide consisting of the amino acid sequence in which one or more amino acid residues are deleted, substituted or added in said amino acid sequence of (a), or
(c) a polypeptide consisting of the amino acid sequence having 75% or higher identity to said amino acid sequence of (a).

In the present invention, the racemization reaction is carried out in an aqueous solution with the concentration of the α-amino acid in the range of 0.1% by weight - saturated concentration and the amount of the enzyme preferably in the range of 0.00001 to 3 times to the weight of α-amino acid as the raw material, more preferably 0.002 to 0.5 times as the basis of dry bacterial cells containing the enzyme. The reaction temperature is preferably in the range of 10 to 70 °C, more preferably 30 to 60 °C with pH preferably in the range of 4 to 13, more preferably 5 to 10, further preferably 6 to 9.

### Method for preparing optimally active α-amino acids

According to another embodiment of the present invention, there is provided a method for racemization of optically active α-amino acids, comprising applying the racemization method according to the present invention to an α-amino acid mixture enriched in either the D-or L-isomer due to optical resolution to give a racemized α-amino acid, from which the desired (aimed) D- or L-isomer is obtained by optical resolution.
In this connection, the method of optical resolution includes, for example, the optical resolution method with a resolving agent described later.

According to another preferred embodiment of the present invention, there is provided a method for racemization of optically active α-amino acids, comprising
making a resolving agent which stereoselectively forms a low solubility salt with either one of the L-α-amino acid represented by the following formula (1) or the D-α-amino acid represented by the following formula (2) to act on the mixture of the D- and L-isomers and separating the either one of the D- or L-amino acid as the solid of a diastereomer salt (optical resolution step);
making the γ-aminobutyric acid aminotransferase according to the present invention, or a viable bacterial cell producing the γ-aminobutyric acid aminotransferase or a processed product thereof to act on the remaining α-amino acid mixture enriched in either the D-or L-isomer to conduct recemization (racemization step); and
subjecting the recemized mixture to the stereoselective resolution by forming a diastereomer salt with a resolving agent again (optical resolution step). Thus, it becomes possible to obtain the desired optically active α-amino acid in a good yield.

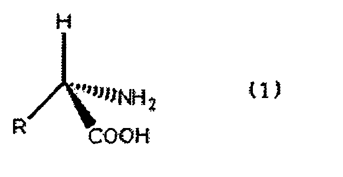

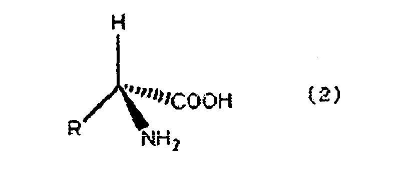

Thus, a new L-isomer is obtained by making a resolving agent salt which stereoselectively forms a low solubility salt together with the L-isomer to act on the mixture of the D- and L-isomers, separating the L-isomer as the solid of a diastereomer salt, making a viable bacterial cell having the racemization activity of an amino acid or the like to act on the remaining D-isomer or mixture of the D- and L-isomers, and subjecting again the resulting mixture enriched in the L-isomer to optical resolution. The L-isomer of the α-amino acid can be obtained from the mixture of the D- and L-isomers by repeating these operations. (In the same manner, the D-isomer of the α-amino acid can be also obtained.)

In this connection, R in the formula (1) or (2) is a benzyl group which is optionally substituted by methyl or hydroxy groups, or a linear or branched alkyl group having 1 to 4 carbon atoms. The alkyl group includes, for example, methyl, ethyl, propyl, isopropyl, n-butyl, sec-butyl groups, and the like, among which the ethyl group is preferred. The alkyl groups may be substituted by hydroxy, methylthio, thiol, amide, or imidazolyl groups.

Specific examples of the optically active α-amino acids include alanine, valine, leucine, isoleucine, tert-leucine, histidine, phenylalanine, methionine, asparagine, tyrosine, serine, 2-aminobutyric acid, and the like.

Moreover, as the mixture of the L-α-amino acid represented by the formula (1) and the D-α-amino acid represented by the formula (2) is used suitably the racemic modification. The mixture may also be a mixture which is enriched in either one of the L- or D-isomer. The mixture may also be a mixture which is obtained by racemizing either one of the L- or D-isomer of the optically active α-amino acid. Furthermore, the racemic mixture of the α-amino acid represented by the formulae (1) and (2) can be obtained, for example, by hydrolyzing an α-aminonitrile which is easily prepared by the Strecker reaction.

A variety of methods are known as the method for optically resolving the α-amino acid, and a method in which either one of the D- or L-isomers as the optical isomers and a resolving agent which can stereoselectively form a low solubility salt are used can be preferably employed in the present invention. According to this method, optical resolution can be carried out by stereoselectively forming a diastereomer salt having a low solubility with a resolving agent, solidifying the salt by taking advantage of the difference of solubilities, and separating the salt by filtration or the like. By way of example, the racemic 2-aminobutyric acid and a half equivalent of (R)-2-phenoxypropionic acid are dissolved by heating into water, followed by cooling and crystallization, thus resulting in L-2-aminobutyric acid/(R)-2-phenoxypropionic acid.

The resolving agent which can be used in the present invention may be appropriately selected depending on the states of the aimed amino acid or the optical isomer, and specifically includes tartaric acid, mandelic acid, 2-phenoxypropionic acid, and the like. 2-phenoxypropionic acids are particularly suitable for the present invention because of easiness in their aftertreatment. The resolving agent may be further used in the next optical resolution again by separating and collecting it after the desired α-amino acid has been obtained.

### EXAMPLES

The present invention is specifically described by examples, but it is not restricted by these examples.

The HPLC analysis condition in the analysis of the reaction solution was described in the following:

| HPLC analysis condition (1) | | |
|---|---|---|
| Eluant | 1 mM CuSO₄ aqueous solution | |
| Flow rate | 1 ml/min | |
| Column | SUMICHIRAL OA-5000 (Sumika Chemical Analysis Service, Ltd.) | |
| Column thermostatic chamber | | 30°C |
| Detector | UV 254 nm | |

| HPLC analysis condition (2) | | |
|---|---|---|
| Eluant | 50 mM HClO₄ aqueous solution | |
| Flow rate | 0.2 ml/min | |
| Column | CROWNPAK CR(+) 5 µm 4.0 x 150 mm (Daicel Corporation) | |
| Column thermostatic chamber | | 30°C |
| Detector | UV 254 nm | |

### Preparation Example 1

A primer was designed on the basis of the base sequence regarded as the γ-aminobutyric acid aminotransferase gene obtained from the genomic DNA information of *Leuconostoc mesenteroides subsp. mesenteroides* ATCC 8293 or *Staphylococcus saprophyticus subsp. saprophyticus* ATCC 15305 (http://gib.genes.nig.ac.jp/).
"The base sequence regarded as the γ-aminobutyric acid aminotransferase gene" herein is the base sequence which is classified into 4-aminobutyrate aminotransferase as the result of the homology search with the algorithm BLAST [Pro. Natl. Acad. Sci. USA, 90, 5873 (1993)]. The specific technique of BLAST is well known (http://www.ncbi.nlm.nih.gov.).

*Leuconostoc mesenteroides subsp. mesenteroides* ATCC 8293 and *Staphylococcus saprophyticus subsp. saprophyticus* ATCC 15305 were cultured in the medium and incubation temperature directed by ATCC, PCR was conducted with the chromosomal DNA extracted therefrom as the template and primers AT8-F and AT8-R (SEQ ID NOS: 9 and 10) or AT15-F and AT15-R (SEQ ID NOS: 11 and 12).

**Table 1**

| Leuconostoc mesenteroides subsp. mesenteroides ATCC 8293 | | |
|---|---|---|
| AT8-F | ctaaaaaagcttgtaggaggtgtagaaaatgtcaaaaataaagagattttagaagaa Hind III                    initiation codon | SEQ ID NO: 9 |
| AT8-R | cgtatgtctagaattcctaatc Xba I | SEQ ID NO: 10 |

| Staphvlococcus saprophyticus subsp. saprophyticus ATCC 15305 | | |
|---|---|---|
| AT15-F | attttaagctttttaggaggcatagattatgaacaaatcagagcaacttataaatgag Hind III initiation codon | SEQ ID NO: 11 |
| AT15-R | gtttatttaaattctagaaggttg Xba I | SEQ ID NO: 12 |

The PCR reaction products obtained were purified, digested with the restriction enzymes Hind III and Xba I and ligated into pUC19 (Takara Shuzo Co., Ltd.) digested with the same enzymes to prepare pAT8 and pAT15, before transformation into E. coli DH5α (obtained from TOYOBO CO., LTD.) by the calcium chloride method. The transformant strain was applied to the LB agar medium containing 50 µg/ml of ampicillin, and the grown up clones were collected to obtain the transformant strains DH5α/pAT8 and DH5α/pAT15.

### Preparation Example 2

A primer was designed on the basis of the base sequence regarded as the γ-aminobutyric acid aminotransferase gene obtained from the genomic DNA information of *Lactobacillus fermentum* IFO 3956, *Lactobacillus reuteri* DSM 20016, *Staphylococcus epidermidis* ATCC 12228, *Leuconostoc citreum* NBRC 102476, or *Pyrococcus horikoshii* OT-3 (http://gib.genes.nig.ac.jpp://gib.genes.nig.ac.jp/) in the same manner as Preparation Example 1.

*Lactobacillus fermentum* IFO 3956, *Lactobacillus reuteri* DSM 20016, *Staphylococcus epidermidis* ATCC 12228, *Leuconostoc citreum* NBRC 102476 or *Pyrococcus horikoshii* OT-3 were cultured in the medium at the incubation temperature directed, PCR was conducted with the chromosomal DNA extracted therefrom as the template and primers AT9-F and AT9-R (SEQ ID NOS: 13 and 14), AT10-F and AT10-R (SEQ ID NOS: 15 and 116), AT11-F and AT11-R (SEQ ID NOS: 17 and 18), AT12-F and AT12-R (SEQ ID NOS: 19 and 20), AT13-F and AT13-R (SEQ ID NOS: 21 and 22) or AT14-F and AT14-R (SEQ ID NOS: 23 and 24).

**TABLE 2**

| Lactobacillus fermentum IFO 3956 | | |
|---|---|---|
| AT9-F | gagacactgcatatggaggagaaaaaaagtaacc Nde I | SEQ ID NO: 13 |
| AT9-R | ctgaattcttattcccaacccagctcttctgcg EcoR I | SEQ ID NO: 14 |

| Lactobacillus reuteri DSM 20016 | | |
|---|---|---|
| AT10-F | gaagatggaccatatgttaaaaacgaaactgaagc Nde I | SEQ ID NO: 15 |
| AT10-R | gattggtcgacttaccaacctaactcttctgcatttg Sal I | SEQ ID NO: 16 |

| Staphylococcus epidermidis ATCC 12228 | | |
|---|---|---|
| AT11-F | gagggaaccatatgagtaaggttaatcatttg Nde I | SEQ ID NO: 17 |
| AT11-R | caggaattcttaccaaccttgaccactaatgtc EcoR I | SEQ ID NO: 18 |

| Leuconostoc citreum NBRC 102476 | | |
|---|---|---|
| AT12-F | ggggcgggacatatgtctaacaataaaaaaattttagc Nde I | SEQ ID NO: 19 |
| AT12-R | gcgaattcttaccagccaatgtgattatccg EcoR I | SEQ ID NO: 20 |

| Pyrococcus horikoshii OT-3 | | |
|---|---|---|
| AT13-F | gttctgctgcaggaggaggattaagagatggtttccatgaccaagtggg Pst I initiation codon | SEQ ID NO: 21 |
| AT13-R | gagtgtgaattcatggggatatgctagagtgt EcoR I | SEQ ID NO: 22 |

| Pyrococcus horikoshii OT-3 | | |
|---|---|---|
| AT14-F | gaatcccatatgccttttctacccttttc Nde I | SEQ ID NO: 23 |
| AT14-R | gaagccgaattcgagcagaagcctgtgatacc EcoR I | SEQ ID NO: 24 |

After the PCR reaction products obtained were purified, the one obtained with the chromosomal DNA of *Lactobacillus fermentum* IFO 3956, *Staphylococcus epidermidis* ATCC 12228 or *Leuconostoc citreum* NBRC 102476 as the template was digested with restriction enzymes NdeI and EcoRI, and the one obtained with the chromosomal DNA of *Lactobacillus reuteri* DSM 20016 as the template was digested with restriction enzymes NdeI and SalI. As regards *Pyrococcus horikoshii* OT-3, the PCR reaction product obtained with the primers AT13-F and AT13-R and the chromosomal DNA as the template was digested with restriction enzymes PstI and EcoRI, and the PCR reaction product obtained with the primers AT14-F and AT14-R and the chromosomal DNA as the template was digested with restriction enzymes NdeI and EcoRI.

pTV118N-NdeI was prepared by introducing the restriction enzyme NdeI cleavage sequence (CATATG) into the position of initiation codon (ATG) of IaqZα on pTV118N (Takara Shuzo Co., Ltd.) with primers NdeF and NdeR (SEQ ID NOS: 25 and 26) and QuikChange^{R} Site-Directed Mutagenesis Kit (Stratagene).

**TABLE 3**

| | | |
|---|---|---|
| NdeF | cacaggaaacagcatatggccatgattacg Nde I | SEQ ID NO: 25 |
| NdeR | cgtaatcatggccatatgctgtttcctgtg Nde I | SEQ ID NO: 26 |

pAT9, pAT10, pAT11, pAT12 and pAT14 were prepared by linking each of the PCR reaction products with AT9-F and AT9-R, AT10-F and AT10-R, AT11-F and AT11-R, AT12-F and AT12-R, and AT14-F and AT14-R to pTV118N-NdeI digested with restriction enzymes NdeI and EcoRI which digested the PCR products. Furthermore, pAT13 was prepared by linking the PCR reaction product with AT13-F and AT13-R to pUC19 digested with restriction enzymes PstI and EcoRI which digested the PCR products. pAT9, pAT10, pAT11, pAT12, pAT13 and pAT14 were transformed into E. coli (DH5α) by the calcium chloride method. The transformant strain was applied to the LB agar medium containing 50 µg/ml of ampicillin, and the grown up clones were collected to obtain the transformant strains DH5α/pAT9, DH5α/pAT10, DH5α/pAT11, DH5α/pAT12, DH5α/pAT13 and DH5α/pAT14.

### Example 1

DH5α/pAT8 prepared in Preparation Example 1 was cultured in 500 ml of LB medium at 37°C for 17 hours. The cells were collected by centrifugation and subjected to the racemization reaction of D-2-aminobutyric acid.
D-2-aminobutyric acid (50 g) was dissolved in distilled water (450 g), and DH5α/pAT8 (0.75 g, corresponding to the dry weight) was added. The mixture was stirred at 50°C for 24 hours and centrifuged for removing bacteria. The reaction was analyzed by HPLC (analysis condition (1)) resulting in the ratio of L-2-aminobutyric acid to D-2-aminobutyric acid 0.99 : 1.

### Example 2

DH5α/pAT15 prepared in Preparation Example 1 was cultured in 500 ml of LB medium at 37°C for 17 hours. The cells were collected by centrifugation and subjected to the racemization reaction of D-2-aminobutyric acid.
D-2-aminobutyric acid (50 g) was dissolved in distilled water (450 g) and DH5α/pAT15 (0.75 g, corresponding to the dry weight) was added after adjusting pH to 8 with sodium hydroxide. The mixture was stirred at 50°C for 24 hours and centrifuged for removing bacteria. The reaction was analyzed by HPLC (analysis condition (1)) resulting in the ratio of L-2-aminobutyric acid to D-2-aminobutyric acid 0.096 : 1.

### Example 3

DH5α/pAT8 and DH5α/pAT15 prepared in Preparation Example 1 were cultured in 200 ml of LB medium at 37°C for 17 hours. The cells were collected by centrifugation, suspended in pure water, fragmented by ultrasonic waves, centrifuged again for removing the residue, and the supernatant was subjected to the racemization reaction.
The supernatant was added to the aqueous solution of 5% D-2-aminobutyric acid (200 ml) adjusted to pH 7.5, and the mixture was stirred at 37°C for 24 hours. The reaction was analyzed by HPLC (analysis condition (1)) resulting in the ratio of L-2-aminobutyric acid to D-2-aminobutyric acid 0.75 : 1 and 0.08 : 1 when using DH5α/pAT8 and DH5α/pAT15, respectively.

### Example 4

DH5α/pAT8 and DH5α/pAT15 prepared in Preparation Example 1 were cultured in 200 ml of LB medium at 37°C for 17 hours. The cells were collected by centrifugation and subjected to the racemization reaction of each amino acid described in Table 4.
To 100 g of each aqueous amino acid solution adjusted to pH 7.5 were suspended 0.25 g, 0.1 g and 0.0015 g (each corresponding to dry weight) of the collected cells having a substrate concentration of 2.5%, 1% and 0.015%, respectively, and the mixtures were stirred at 37°C for 24 hours. The reactions were analyzed by HPLC (analysis condition (2)).
The results are shown in Table 4.

**TABLE 4**

| Substrate amino acid Substrate concentration | | pAT8 | pAT15 |
|---|---|---|---|
| L-Phenylalanine | 2.5% | 0.998 | 0.249 |
| L-2-aminobutyric acid | 2.5% | 0.993 | 0.046 |
| L-Valine | 2.5% | 1 | 0.225 |
| L-Alanine | 2.5% | 1 | 0.073 |
| L-Serine | 2.5% | 0.590 | 0 |
| L-Isoleucine | 2.5% | 1 | 1 |
| L-Histidine | 2.5% | 0.175 | 0 |
| L-tert-Leucine | 2.5% | 0.023 | 0.009 |
| L-Methionine | 1.0% | 1 | 0.022 |
| L-Leucine | 1.0% | 0.979 | 0.319 |
| L-Asparagine | 1.0% | 0.115 | 0.260 |
| L-Tyrosine | 0.015% | 0.017 | 0.001 |

| | | | |
|---|---|---|---|
| (Values: D-amino acid/L-amino acid) | | | |

### Example 5

DH5α/pAT9, DH5α/pAT10 or DH5α/pAT11 prepared in Preparation Example was cultured in 200 ml of LB medium at 37°C for 17 hours. After collecting the cells by centrifugation, the cells were subjected to the racemization reaction of D-2-aminobutyric acid.
To 200 ml of 10% aqueous D-2-aminobutyric acid solution adjusted to pH 7 was suspended 0.2 g (corresponding to dry weight) of the collected cells, pyridoxal-5'-phosphate (PLP) in the final concentration of 40 µM was added, and the mixture was stirred at 40°C for 24 hours. HPLC analysis (analysis condition (1)) of the reaction resulted in the ratios of L-2-aminobutyric acid to D-2-aminobutyric acid described in the following:
when DH5α/pAT9 was used, 0. 053 : 1;
when DH5α/pAT10 was used, 0. 965 : 1; and
when DH5α/pAT11 was used, 0.067 : 1.

### Example 6

DH5α/pAT12 prepared in Preparation Example was cultured in 200 ml of LB medium at 30°C for 17 hours. After collecting the cells by centrifugation, the cells were subjected to the racemization reaction of D-2-aminobutyric acid.
To 200 ml of 10% aqueous D-2-aminobutyric acid solution adjusted to pH 7 was suspended 0.2 g (corresponding to dry weight) of the collected cells, PLP in the final concentration of 40 µM was added, and the mixture was stirred at 40°C for 24 hours. HPLC analysis (analysis condition (1)) of the reaction resulted in the ratio of L-2-aminobutyric acid to D-2-aminobutyric acid 1:1.

### Example 7

DH5α/pAT13 and DH5α/pAT14 prepared in Preparation Example were cultured in 200 ml of LB medium at 37°C for 17 hours. After collecting the cells by centrifugation, the cells were subjected to the racemization reaction of D-2-aminobutyric acid.
To 100 ml of 1 % aqueous D-2-aminobutyric acid solution adjusted to pH 7 was suspended 0.1 g (corresponding to dry weight) of the collected cells, and the mixture was stirred at 70°C for 24 hours. HPLC analysis (analysis condition (1)) of the reaction resulted in the ratios of L-2-aminobutyric acid to D-2-aminobutyric acid described in the following:
when DH5α/pAT13 was used, 0. 360 : 1; and
when DH5α/pAT14 was used, 0.078 : 1.

### Example 8: Method for preparing an α-amino acid

### Step 1

Operations were performed according to the method described in Japanese Patent Laid-Open Publication No. 2006-169158. Specifically, racemic 2-aminobutyric acid (100 g), (R)-2-phenoxypropionic acid (80 g, equimolar amount to L-2-aminobutyric acid) and distilled water (900 g) were mixed and stirred overnight. The solution was cooled to a temperature of 20 to 30°C, and the resulting crystals were filtered, rinsed with distilled water and dried to give the diastereomeric crystals (110.5 g). L-2-aminobutyric acid in the crystals had an optical purity of 99% ee or more on the basis of HPLC.

### Step 2

To the diastereomeric crystals (50 g) obtained in the step 1 were added ethyl acetate (200 ml) and water (200 ml), and the mixture was stirred at room temperature for 2 hours and then separated. To the aqueous layer was added ethyl acetate (200 ml), and the mixture was stirred at room temperature for 2 hours. The aqueous layer was concentrated to give L-2-aminobutyric acid in an amount of 17.2 g (yield, 95%). L-2-aminobutyric acid thus obtained had an optical purity of 99% ee or more on the basis of HPLC.
In this connection, the ethyl acetate layer was concentrated, and (R)-2-phenoxypropionic acid was collected quantitatively.

### Step 3

DH5α/pAT8 prepared in Preparation Example 1 was cultured in 500 ml of LB medium at 37°C for 17 hours. After collecting the cells by centrifugation, the cells were subjected to the racemization reaction described below.
The filtrate obtained in the step 1 was concentrated, suspended in 50% aqueous acetone solution, and stirred at room temperature for 2 hours. After filtration and drying, the crystals (50 g) obtained was dissolved in distilled water (450 g), and the collected DH5α/pAT8 was added. The mixture was stirred at 40°C for 24 hours, and centrifuged for removing bacteria. The reaction was analyzed by HPLC resulting in the ratio of L-2-aminobutyric acid to D-2-aminobutyric acid 0.99 : 1.

### Step 4

To an aqueous 2-aminobutyric acid solution obtained in the step 3, (R)-2-phenoxypropionic acid (40 g, equimolar amount to L-2-aminobutyric acid) was mixed and stirred overnight. The solution was cooled to a temperature of 20 to 30°C, and the resulting crystals were filtered, rinsed and dried to give the diastereomeric crystals (55.3 g). L-2-aminobutyric acid in the crystals had an optical purity of 99% ee or more on the basis of HPLC.

### Examples 9 to 12

Operations were practiced in the same manner as Example 8, except DH5α/pAT8 in the step 3 was replaced with DH5α/pAT9, DH5α/pAT10, DH5α/pAT11 prepared in Preparation Example 2 or with DH5α/pAT15 prepared in Preparation Example 1.
The filtrate obtained in the step 1 was concentrated, suspended into 50% aqueous acetone solution, and stirred at room temperature for 2 hours. After filtration and drying, the crystals (50 g) obtained were dissolved in distilled water (450 g), and the collected DH5α/pAT9, DH5α/pAT10, DH5α/pAT11 or DH5α/pAT15 was added. The mixture was stirred at 40°C for 24 hours, and centrifuged for removing bacteria.

The results of the HPLC analysis of the reactions are shown in Table 5.
The reactions (2-aminobutyric acid aqueous solutions) were subjected to optical resolution in the same manner as the step 4 in Example 8. All of the L-2-aminobutyric acids in the crystals obtained had an optical purity of 99% ee or more on the basis of HPLC.

**TABLE 5**

| | Bacteria | L-ABA/DABA |
|---|---|---|
| Example 2 | DH5α/pAT9 | 0.058 |
| Example 3 | DH5α/pAT10 | 0.964 |
| Example 4 | DH5a/pAT11 | 0.033 |
| Example 5 | DH5α/pAT12 | 0.108 |

| | | |
|---|---|---|
| L-ABA: L-2-aminobutyric acid; D-ABA: D-2-aminobutyric acid. | | |

### Example 13

Operations were practiced in the same manner as Example 8, except DH5α/pAT8 in the step 3 was replaced with DH5α/pAT12 prepared in Preparation Example 2 and culture was carried out at a temperature of 30°C.
The filtrate obtained in the step 1 was concentrated, suspended into 50% aqueous acetone solution and stirred at room temperature for 2 hours. After filtration and drying, the crystals (50 g) obtained were dissolved in distilled water (450 g), pyridoxal-5'-phosphate (PLP) in the final concentration of 100 µM was added, and the collected DH5α/pAT12 was added. The mixture was stirred at 40°C for 24 hours, and centrifuged for removing bacteria. HPLC analysis of the reaction resulted in the ratio of L-2-aminobutyric acid to D-2-aminobutyric acid 1 : 1.
The reaction (2-aminobutyric acid aqueous solution) was subjected to optical resolution in the same manner as the step 4 in Example 1. L-2-aminobutyric acids in the crystals obtained had an optical purity of 99% ee or more on the basis of HPLC.

### Examples 14 and 15

Operations were practiced in the same manner as Example 8, except DH5α/pAT8 in the step 3 was replaced with DH5α/pAT13 or DH5α/pAT14 prepared in Preparation Example 2.
The filtrate obtained in the step 1 was concentrated, suspended into 50% aqueous acetone solution, and stirred at room temperature for 2 hours. After filtration and drying, the crystals (50 g) obtained were dissolved in distilled water (450 g), and the collected DH5α/pAT13 or DH5α/pAT14 was added. The mixture was stirred at 40°C for 96 hours, and centrifuged for removing bacteria. HPLC analysis of the reaction resulted in the ratio of L-2-aminobutyric acid to D-2-aminobutyric acid 0.445 : 1 and 0.248 : 1 when using DH5α/pAT13 and DH5α/pAT14, respectively.
The reaction (2-aminobutyric acid aqueous solution) was subjected to optical resolution in the same manner as the step 4 in Example 1. L-2-aminobutyric acids in the diastereomeric crystals obtained had an optical purity of 99% ee or more on the basis of HPLC.

## Claims

1. A method for racemization of an optically active α-amino acid, comprising racemizing an optically active α-amino acid with γ-aminobutyric acid aminotransferase producing viable bacterial cells derived from genera *Leuconostoc, Lactobacillus, Weissella, Oenococcus, Staphylococcus, Aminobacterium, Alkaliphilus, Clostridium, Pyrococcus, Thermococcus, Thermofilum, Pyrobaculum, Anoxybacillus, Syntrophus, Thermoproteus, Symbiobacterium, Desulfurococcus, Bacillus, Acidobacteria, Clostridium, Deinococcus, Pelobacter, Geobacillus, Geobacter, Pelotomaculum, Solibacter, Coprothermobacter, Aeropyrum, Gloeobacter, Nostoc, Herpetosiphon, Metallosphaera, Syntrophobacter, Photorhabdus, Caldivirga, Hyperthermus, Thermoplasma, Gemmata, Anabaena, Cyanothece, Picrophilus* or *Chloroflexus,* or a processed product thereof.

2. A method according to claim 1, wherein γ-aminobutyric acid aminotransferase is derived from bacteria belonging to:
*Leuconostoc mesenteroides subsp. mesenteroides,*
*Staphylococcus saprophyticus subsp. saprophyticus,*
*Lactobacillus fermentum,*
*Lactobacillus reuteri,*
*Staphylococcus epidermidis,*
*Leuconostoc citreum,* or
*Pyrococcus horikoshii.*

3. A method according to claims 1 or 2, wherein γ-aminobutyric acid aminotransferase is derived from:
*Leuconostoc mesenteroides subsp. mesenteroides* ATCC 8293,
*Staphylococcus saprophyticus subsp. saprophyticus* ATCC 15305,
*Lactobacillus fermentum* IFO 3956,
*Lactobacillus reuteri* DSM 20016,
*Staphylococcus epidermidis* ATCC 12228,
*Leuconostoc citreum* NBRC 102476, or
*Pyrococcus horikoshii* OT-3.

4. A method according to any one of claims 1 to 3, wherein γ-aminobutyric acid aminotransferase is a polypeptide selected from:
(a) a polypeptide consisting of an amino acid sequence selected from the group consisting of the amino acid sequences of SEQ ID NOS: 1, 2, 3, 4, 5, 6, 7 and 8,
(b) a polypeptide consisting of the amino acid sequence in which one or more amino acid residues are deleted, substituted or added in said amino acid sequence of (a), or
(c) a polypeptide comprising the amino acid sequence having 75% or higher identity to said amino acid sequence of (a).

5. A method for racemization of optically active α-amino acids, comprising racemizing optically active α-amino acids with γ-aminobutyric acid aminotransferase selected from:
(a) a polypeptide consisting of an amino acid sequence selected from the group consisting of the amino acid sequences of SEQ ID NOS: 1, 2, 3, 4, 5, 6, 7 and 8,
(b) a polypeptide consisting of the amino acid sequence in which one or more amino acid residues are deleted, substituted or added in said amino acid sequence of (a), or
(c) a polypeptide consisting of the amino acid sequence having 75% or higher identity to said amino acid sequence of (a).

6. A method according to any one of claims 1 to 5, wherein γ-aminobutyric acid aminotransferase is a polypeptide derived from *Leuconostoc mesenteroides subsp. mesenteroides* ATCC 8293 and selected from:
(a1) a polypeptide consisting of an amino acid sequence selected from the group consisting of the amino acid sequence of SEQ ID NO: 1,
(b1) a polypeptide consisting of the amino acid sequence in which one or more amino acid residues are deleted, substituted or added in said amino acid sequence of (a1), or
(c1) a polypeptide consisting of the amino acid sequence having 75% or higher identity to said amino acid sequence of (a1).

7. A method according to any one of claims 1 to 5, wherein γ-aminobutyric acid aminotransferase is a polypeptide derived from *Staphylococcus saprophyticus subsp. saprophyticus* ATCC 15305 and selected from:
(a2) a polypeptide consisting of the amino acid sequence selected from the group consisting of the amino acid sequence of SEQ ID NO:2,
(b2) a polypeptide consisting of the amino acid sequence in which one or more amino acid residues are deleted, substituted or added in said amino acid sequence of (a2), or
(c2) a polypeptide consisting of the amino acid sequence having 75% or higher identity to said amino acid sequence of (a2).

8. A method according to any one of claims 1 to 5, wherein γ-aminobutyric acid aminotransferase is a polypeptide derived from *Lactobacillus fermentum* IFO 3956 and selected from:
(a3) a polypeptide consisting of an amino acid sequence selected from the group consisting of the amino acid sequence of SEQ ID NO: 3,
(b3) a polypeptide consisting of the amino acid sequence in which one or more amino acid residues are deleted, substituted or added in said amino acid sequence of (a3), or
(c3) a polypeptide consisting of the amino acid sequence having 75% or higher identity to said amino acid sequence of (a3).

9. A method according to any one of claims 1 to 5, wherein γ-aminobutyric acid aminotransferase is a polypeptide derived from *Lactobacillus reuteri* DSM 20016 and selected from:
(a4) a polypeptide consisting of an amino acid sequence selected from the group consisting of the amino acid sequence of SEQ ID NO: 4,
(b4) a polypeptide consisting of the amino acid sequence in which one or more amino acid residues are deleted, substituted or added in said amino acid sequence of (a4), or
(c4) a polypeptide consisting of the amino acid sequence having 75% or higher identity to said amino acid sequence of (a4).

10. A method according to any one of claims 1 to 5, wherein γ-aminobutyric acid aminotransferase is a polypeptide derived from *Staphylococcus epidermidis* ATCC 12228 and selected from:
(a5) a polypeptide consisting of an amino acid sequence selected from the group consisting of the amino acid sequence ID NO: 5,
(b5) a polypeptide consisting of the amino acid sequence in which one or more amino acid residues are deleted, substituted or added in said amino acid sequence of (a5), or
(c5) a polypeptide consisting of the amino acid sequence having 75% or higher identity to said amino acid sequence of (a5).

11. A method according to any one of claims 1 to 5, wherein γ-aminobutyric acid aminotransferase is a polypeptide derived from *Leuconostoc citreum* NBRC 102476 and selected from:
(a6) a polypeptide consisting of an amino acid sequence selected from the group consisting of the amino acid sequence of SEQ ID NO: 6,
(b6) a polypeptide consisting of the amino acid sequence in which one or more amino acid residues are deleted, substituted or added in said amino acid sequence of (a6), or
(c6) a polypeptide consisting of the amino acid sequence having 75% or higher identity to said amino acid sequence of (a6).

12. A method according to any one of claims 1 to 5, wherein γ-aminobutyric acid aminotransferase is a polypeptide derived from *Pyrococcus horikoshii* OT-3 and selected from:
(a7) a polypeptide consisting of an amino acid sequence selected from the group consisting of the amino acid sequences of SEQ ID NOS: 7 or 8,
(b7) a polypeptide consisting of the amino acid sequence in which one or more amino acid residues are deleted, substituted or added in said amino acid sequence of (a7), or
(c7) a polypeptide consisting of the amino acid sequence having 75% or higher identity to said amino acid sequence of (a7).

13. A method according to any one of claims 1 to 12, wherein the optically active α-amino acid comprises one or more of the compounds represented by the following formula (I): 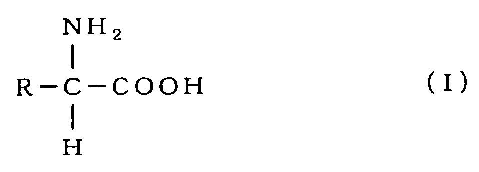 wherein
R represents
a benzyl group which is optionally substituted by methyl or hydroxy groups, or
a linear or branched alkyl group having 1 to 4 carbon atoms, wherein the alkyl group is optionally substituted by hydroxy, methylthio, thiol, amide, or imidazolyl groups.

14. A method according to claim 13, wherein optically active α-amino acid comprises one or more of the compounds selected from the group consisting of alanine, valine, leucine, isoleucine, tert-leucine, histidine, phenylalanine, methionine, asparagine, tyrosine, serine and 2-aminobutyric acid.

15. A method according to any one of claims 1 to 14, wherein the optically active α-amino acid is the L form.

16. A method according to any one of claims 1 to 14, wherein the optically active α-amino acid is the D form.
